# EUROPEAN PATENT SPECIFICATION

(11) **EP 3 578 162 B1**
(45) Date of publication and mention of the grant of the patent: **26.06.2024**
(21) Application number: 17894930.1
(22) Date of filing: 31.01.2017
(51) Int. Cl.: A61K 8/34, A61K 8/36, A61Q 5/04, A61K 8/362, A61K 8/44, A61K 8/46

(54) **HAIR TREATMENT METHOD**
HAARBEHANDLUNGSVERFAHREN
MÉTHODE DE TRAITEMENT CAPILLAIRE

(43) Date of publication of application: 11.12.2019
(73) Proprietor: Kao Corporation, Chuo-Ku Tokyo 103-8210 (JP)
(72) Inventor: NAGASAKI, Ayaka, Tokyo 131-8501 (JP); NIWANO, Yu, Tokyo 131-8501 (JP); ITAYA, Miki, Tokyo 131-8501 (JP)
(74) Representative: Hoffmann Eitle
(86) International application number: PCT/JP2017/003463
(87) International publication number: WO 2018/142484

(56) References cited:
- EP-A1- 2 314 278
- EP-A1- 2 520 274
- WO-A1-02/098381
- WO-A1-2013/174575
- DE-A1- 19 536 423
- JP-A- 2003 321 332
- JP-A- 2006 282 613
- JP-A- 2006 315 978
- JP-A- 2008 013 549
- JP-A- 2008 024 609
- JP-A- 2009 137 886
- JP-A- 2015 030 718
- US-A1- 2004 011 373
- DATABASE GNPD [Online] MINTEL; 4 July 2006 (2006-07-04), anonymous: "Straight Treatment", XP055707078, retrieved from www.gnpd.com Database accession no. 554135
- DATABASE GNPD [Online] MINTEL; 31 March 2011 (2011-03-31), anonymous: "Quick Blow Mist", XP055707082, retrieved from www.gnpd.com Database accession no. 1549519
- DATABASE GNPD [Online] MINTEL; 9 July 2015 (2015-07-09), anonymous: "Straightening Shampoo", XP055707088, retrieved from www.gnpd.com Database accession no. 3026297
- DATABASE GNPD [Online] MINTEL; 7 November 2016 (2016-11-07), anonymous: "Shampoo & Conditioner Set", XP055707089, retrieved from www.gnpd.com Database accession no. 4390667

## Description

### Field of the Invention

The present invention relates to a hair treatment method.

### Background of the Invention

Conventionally, a treatment called a straight perm treatment or a relaxer treatment have been usually employed when wishing relaxing or straightening curly hair. In the straight perm treatment, curly hair is forcibly straightened by cleaving a disulfide bond present in a keratin protein within hair by a reducing agent such as thioglycolic acid, if necessary, with the help of a tool such as a high-temperature hair iron, and thereafter, a treatment with an oxidizing agent such as hydrogen peroxide and potassium bromate is applied to the hair to re-form the disulfide bond. In the relaxer treatment, hair is forcibly straightened (deformed/fixed) by cleaving a disulfide bond of a hair protein by a strong alkali (pH 12 to 14) hair treatment agent and substituting the bond with a lanthionine bond.

However, these methods are known to sometimes seriously damage hair since a strong chemical reaction and high temperature heat are applied. Therefore, various investigations have been made in order to develop a method of straightening curly hair and managing hair without heating and without applying neither a straight perm treatment nor a relaxer treatment.

For example, in Patent Document 1, it is disclosed that wavy/curly hair is easily straightened, without damaging, by applying an acidic hair straightening-agent composition, which contains a compound selected from the group consisting of pyroglutamic acid, hydroxycarboxylic acid, and dicarboxylic acid and a penetration accelerator such as benzyl alcohol, to wavy/curly hair and warming the hair at 40°C for one hour, and that the treated hair has excellent durability to high humidity.

In Patent Document 2, it is disclosed that hair is straightened by applying a composition containing 4 wt% or more of a bidentate or tridentate carboxylic acid and having pH of 3 or less, to dry hair and leaving the hair for 20 minutes.

In Patent Document 3, it is disclosed that wavy/curly hair can be easily straightened, without damaging, by applying an acidic composition containing certain carboxylic acid, certain sulfonic acid, and an organic solvent to the wavy/curly hair and warming the hair at 40°C for one hour, and that the straightening effect is not lost by humidity or shampooing.

Patent Document 4 discloses a technology for providing softness, shine, and manageability to hair simultaneously by applying a hair composition containing a certain compound having 2 or 3 amino acid residues and an aliphatic carboxylic acid having 8 or less carbon atoms or a salt thereof, to the hair.

Patent Document 5 describes a hair treatment process comprising step A or step B, wherein in step A, a first pack with a reducing agent and a second pack with an oxidizing agent are successively applied to the hair, and in step B, a first pack with a reducing agent, an intermediate treatment having a buffer capacity and a specific pH and a second pack with an oxidizing agent are successively applied to the hair.

Patent Document 6 describes a method of straightening hair comprising the application of a hair treatment composition having a specific pH and containing a bidentate or tridentate carboxylic acid.

Patent Document 7 describes a hair cosmetic of non-rinsing-off type comprising a toluenesulfonic acid or a salt thereof, an organic acid or salt thereof and an organic solvent.

(Patent Document 1) JP-A-6-298629
(Patent Document 2) JP-A-2015-517539
(Patent Document 3) JP-A-8-92043
(Patent Document 4) JP-A-2010-65022
(Patent Document 5) US 2004/0011373 A1
(Patent Document 6) WO 2013/174575 A1
(Patent Document 7) JP-A-2008-024609

### Summary of the Invention

In a first aspect, the present invention provides a hair treatment method comprising the following steps (i) to (iii):
step (i): a step of applying a hair treatment agent containing the following components (A) and (B) to hair:
   (A) at least one compound selected from the group consisting of a glycylglycine derivative represented by formula (1) or a salt thereof, a polyglycerin having an average degree of polymerization of 2 or more and 6 or less, and an alkyl glyceryl ether having an alkyl group having 6 or more and 40 or less carbon atoms, 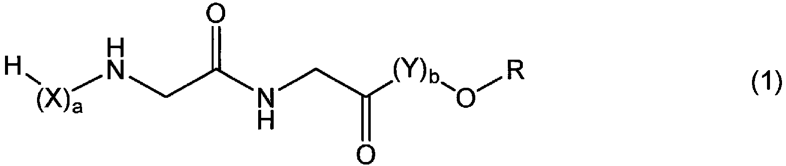
      wherein X represents a divalent hydrocarbon group having 1 to 4 carbon atoms and optionally substituted with hydroxyl group(s), or an amino acid residue, and
      Y represents an amino acid residue or a divalent group represented by formula (2): 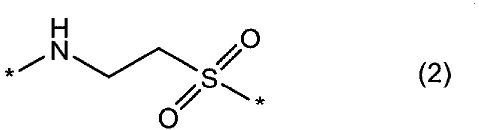
      wherein -* represents a bond to an adjacent carbonyl group or an oxygen atom,
      R represents a hydrogen atom or a monovalent hydrocarbon group having 1 to 4 carbon atoms and optionally substituted with hydroxyl group(s), and
      a and b represent 0 or 1, with the proviso that if a and b simultaneously represent 1, then X does not represent an amino acid residue;
   (B) a carboxylic acid having an inorganic value of 250 or more and 450 or less and an organic value of 50 or more and 250 or less, or a salt of the carboxylic acid, wherein the carboxylic acid is selected from the group consisting of a carboxylic acid represented by the following formula (B-1), malic acid, succinic acid, and lactic acid 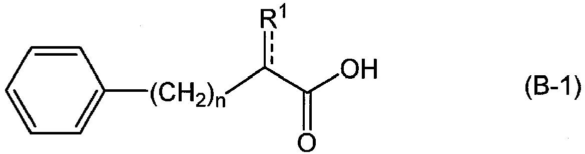 wherein
      R¹ represents a hydrogen atom, an oxygen atom, or a hydroxyl group;
      when R¹ is an oxygen atom, the dashed line indicates a double bond;
      n represents an integer from 0 - 3; and
      the phenyl group and methylene chain can be partly substituted with one or more hydroxyl groups;
step (ii): a step of leaving the hair at 15°C or more and 100°C or less for 15 seconds or more and 60 minutes or less, after step (i); and
step (iii): a step of applying a hair treatment agent containing the following component (C) to the hair, after step (ii) :
   (C) an aromatic sulfonic acid having a molecular weight of 300 or less, or a salt thereof,
wherein the method does not comprise applying a hair treatment agent containing a reducing agent.

In a second aspect, the present invention provides a hair treatment method comprising the following steps (i-a) to (iv'):
step (i-a): a step of applying a one-part hair treatment agent of pH 2 or more and 7.5 or less containing the following components (A), (B), and (D) and substantially not containing a hair reducing agent:
   (A) 1 mass% or more and 10 mass% or less of at least one compound selected from the group consisting of glycylglycine or a salt thereof, glycylglycylglycine or a salt thereof, a polyglycerin having an average degree of polymerization of 2 or more and 4 or less and an alkyl glyceryl ether having an alkyl group having 8 or more and 18 or less carbon atoms
   (B) 1 mass% or more and 15 mass% or less of a carboxylic acid having an inorganic value of 260 or more and 400 or less and an organic value of 60 or more and 180 or less, or a salt of the carboxylic acid, wherein the carboxylic acid is selected from the group consisting of a carboxylic acid represented by the following formula (B-1), malic acid, succinic acid, and lactic acid 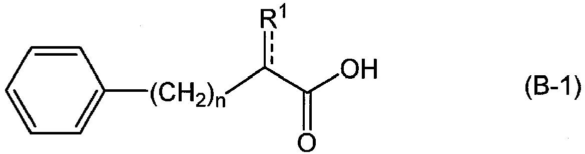 wherein
      R¹ represents a hydrogen atom, an oxygen atom, or a hydroxyl group;
      when R¹ is an oxygen atom, the dashed line indicates a double bond;
      n represents an integer from 0 - 3; and
      the phenyl group and methylene chain can be partly substituted with one or more hydroxyl groups;
   (D) 0.5 mass% or more and 3 mass% or less of a thickener
step (ii): a step of leaving the hair to stand at 15°C or more and 60°C or less for 15 seconds or more and 45 minutes or less, after step (i-a);
step (iii): a step of applying a hair treatment agent of pH 3 or more and 8 or less containing the following components (C), (D), and (E) and water and substantially not containing a hair reducing agent, to the hair, after step (ii):
   (C) 1 mass% or more and 15 mass% or less of at least one selected from the group consisting of 2-naphthalene sulfonic acid, 1-naphthalene sulfonic acid, p-toluenesulfonic acid, xylene sulfonic acid, hydroxymethoxy benzophenone sulfonic acid, and salts of these
   (D) 0.5 mass% or more and 3 mass% or less of a thickener
   (E) 4 mass% or more and 15 mass% or less of an aromatic alcohol;
step (iv): a step of leaving the hair to stand at 15°C or more and 60°C or less for 3 minutes or more and 45 minutes or less, after step (iii);
step (iv'): a step of rinsing the hair, after step (iv).

In a third aspect, the present invention provides a hair treatment method comprising the following steps (i-b) to (iv'):
step (i-b): a step of mixing a first part and a second part of a multi-part hair treatment agent, which contains following component (A) as the first part and following component (B) as the second part or which contains the following component (A) as the second part and the following component (B) as the first part, which contains following component (D) in the first part and/or the second part, and which does not substantially contain a hair reducing agent, and applying the resultant mixture having pH 2 or more and 7.5 or less to hair:
   (A) 1 mass% or more and 10 mass% or less of at least one compound selected from the group consisting of glycylglycine or a salt thereof, glycylglycylglycine or a salt thereof, a polyglycerin having an average degree of polymerization of 2 or more and 4 or less, and an alkyl glyceryl ether having an alkyl group having 8 or more and 18 or less carbon atoms
   (B) 1 mass% or more and 15 mass% or less of a carboxylic acid having an inorganic value of 260 or more and 400 or less and an organic value of 60 or more and 180 or less, or a salt of the carboxylic acid, wherein the carboxylic acid is selected from the group consisting of a carboxylic acid represented by the following formula (B-1), malic acid, succinic acid, and lactic acid 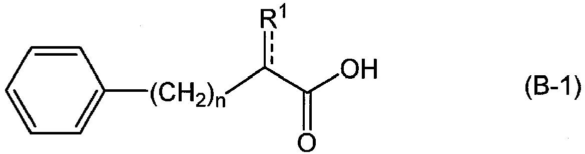 wherein
      R¹ represents a hydrogen atom, an oxygen atom, or a hydroxyl group;
      when R¹ is an oxygen atom, the dashed line indicates a double bond;
      n represents an integer from 0 - 3; and
      the phenyl group and methylene chain can be partly substituted with one or more hydroxyl groups
   (D) 0.5 mass% or more and 3 mass% or less of a thickener,
step (ii): a step of leaving the hair to stand at 15°C or more and 60°C or less for 15 seconds or more and 45 minutes or less, after step (i-b);
step (iii): a step of applying a hair treatment agent of pH 3 or more and 8 or less containing the following components (C), (D), (E), and water and substantially not containing a hair reducing agent to the hair, after step (ii):
   (C) 1 mass% or more and 15 mass% or less of at least one selected from the group consisting of 2-naphthalene sulfonic acid, 1-naphthalene sulfonic acid, p-toluenesulfonic acid, xylene sulfonic acid, hydroxymethoxy benzophenone sulfonic acid, and salts of these
   (D) 0.5 mass% or more and 3 mass% or less of a thickener;
   (E) 4 mass% or more and 15 mass% or less of an aromatic alcohol;
step (iv): a step of leaving the hair to stand at 15°C or more and 60°C or less for 3 minutes or more and 45 minutes or less, after step (iii);
step (iv'): a step of rinsing the hair after step (iv).

In a fourth aspect, the present invention provides a hair treatment method comprising the following steps (i-c-1) to (iv'):
step (i-c-1): a step of applying a first part of a sequential-application type multi-part hair treatment agent of pH 2 or more and 7.5 or less containing the following component (A) and substantially not containing a hair reducing agent, to hair:
   (A) 1 mass% or more and 10 mass% or less of at least one compound selected from the group consisting of glycylglycine or a salt thereof, glycylglycylglycine or a salt thereof, a polyglycerin having an average degree of polymerization of 2 or more and 4 or less, and an alkyl glyceryl ether having an alkyl group having 8 or more and 18 or less carbon atoms;
step (i-cx): a step of leaving the hair to stand at 15°C or more and 60°C or less for 15 seconds or more and 30 minutes or less, after step (i-c);
step (i-c-2): a step of applying a second part of the sequential-application type multi-part hair treatment agent of pH 3.5 or more and 8 or less containing the following components (B) and (D) and substantially not containing a hair reducing agent, after step (i-cx):
   (B) 1 mass% or more and 15 mass% or less of a carboxylic acid having an inorganic value of 260 or more and 400 or less and an organic value of 60 or more and 180 or less, or a salt of the carboxylic acid, wherein the carboxylic acid is selected from the group consisting of a carboxylic acid represented by the following formula (B-1), malic acid, succinic acid, and lactic acid 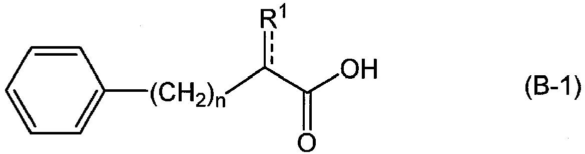 wherein
      R¹ represents a hydrogen atom, an oxygen atom, or a hydroxyl group;
      when R¹ is an oxygen atom, the dashed line indicates a double bond;
      n represents an integer from 0 - 3; and
      the phenyl group and methylene chain can be partly substituted with one or more hydroxyl groups
   (D) 0.5 mass% or more and 3 mass% or less of a thickener
step (ii): a step of leaving the hair to stand at 15°C or more and 60°C or less for 15 seconds or more and 45 minutes or less, after step (i-c-2);
step (iii): a step of applying a hair treatment agent of pH 3 or more and 8 or less containing the following components (C), (D), (E), and water and substantially not containing a hair reducing agent, after step (ii):
   (C) 1 mass% or more and 15 mass% or less of at least one selected from the group consisting of 2-naphthalene sulfonic acid, 1-naphthalene sulfonic acid, p-toluenesulfonic acid, xylene sulfonic acid, hydroxymethoxy benzophenone sulfonic acid, and salts of these
   (D) 0.5 mass% or more and 3 mass% or less of a thickener
   (E) 4 mass% or more and 15 mass% or less of an aromatic alcohol;
step (iv): a step of leaving the hair to stand at 15°C or more and 60°C or less for 3 minutes or more and 45 minutes or less, after step (iii);
step (iv'): a step of rinsing the hair, after step (iv).

### Detailed Description of the Invention

The methods of Patent Documents 1 to 3 was not effective because the curly hair was not sufficiently relaxed in some cases and, in other cases, the state of hair is returned to an untreated curly state after repeated shampooing of hair. Particularly, in Patent Documents 1 and 3, when a simple treatment in which the hair was treated at room temperature for a short time is employed, no effect was obtained.

Hydroxycarboxylic acid such as malic acid is known to provide elasticity and stiffness, and hardness (for example, JP-A-2004-189727) and an aromatic sulfonic acid such as naphthalene sulfonic acid is also known as a compound providing elasticity and stiffness to hair (for example, JP-A-05-229919, JP-A-8-198732). Actually, the treated hair by any one of the methods of Patent Documents 1 to 3 was insufficient in view of softness of the treated hair. Also, any one of the methods of Patent Documents 1 to 3, if it was applied to thin hair like Caucasian hair, had problems of easy-to-detangle and difficult-to-manage.

The method of Patent Document 4 successfully provides softness and manageability to hair, but fails to relax curly hair. Even if this method is used in combination with the methods of Patent Documents 1 to 3, relaxation of curly hair cannot be provided simultaneously with providing softness and manageability.

Accordingly, the present invention relates to a hair treatment method which can relax curly hair, allows hair strands of relaxed curly hair to flow evenly, provides manageable hair, and enables the shape of the hair after the treatment to be maintained even if hair is repeatedly shampooed, and which further provides softness and natural hair smoothness and touch feeling and less detangling of hair, after treatment.

The present inventors conducted intensive studies and found that a curly-hair relaxation effect can be dramatically improved and hair strands evenly flowing can be obtained by treating hair sequentially with a certain glycylglycine derivative, a certain carboxylic acid, and an aromatic sulfonic acid in a predetermined order even if hair treatment time is short; and that the relaxation effect hardly changes even if hair is repeatedly shampooed. Surprisingly, they further found that if hair is treated in a predetermined order, hair smoothness and less detangling as well as softer feeling can be obtained, after treatment. Based on these findings, the present invention has been completed.

The hair treatment method of the present invention can relax curly hair. The relaxed curly hair has strands evenly flowing and is manageable; and the shape of hair just after the treatment can be maintained even if the hair is repeatedly shampooed. Further, the hair treated as above can be a soft, naturally smooth, and less detangled hair.

### • Hair treatment agent used in step (i)

### [Embodiment of hair treatment agent]

In step (i), a hair treatment agent containing components (A) and (B) is used. The hair treatment agent containing components (A) and (B) is constituted of a single composition containing components (A) and (B) or has a composition containing component (A) and a composition containing component (B) as constituents.

If a hair treatment agent is constituted of a composition containing components (A) and (B), an embodiment of the hair treatment agent is specified as one-part hair treatment agent. This embodiment is preferable in order to reduce the treatment time. In contrast, if a hair treatment agent has two compositions; i.e., a composition containing component (A) and a composition containing component (B), as constituents, an embodiment of the hair treatment agent is specified as a multi-part hair treatment agent having a first part containing component (A) and a second part containing component (B) or a multi-part hair treatment agent having a first part containing component (B) and a second part containing component (A). Further, the multi-part hair treatment agent includes a single-application type multi-part hair treatment agent (two compositions are mixed before applying the agent to hair) and a sequential-application type multi-part hair treatment agent (two compositions are sequentially applied to hair).

In the following description, "the composition containing component (A) and/or component (B)" collectively refers to a "composition containing component (A)", a "composition containing component (B)", and a composition "containing components (A) and (B)". The contents of components of the "composition containing component (A)" and the "composition containing component (B)" shall apply to those of the "composition containing components (A) and (B)".

Note that, in the case of the single application multi-part hair treatment agent, since substantially the one step hair treatment agent is applied to hair, the contents of components are referred to those in a composition obtained by mixing a first part and a second part.

### [Component (A): Glycylglycine derivative or a salt thereof, polyglycerin, alkyl glyceryl ether]

Component (A) is at least one compound selected from the group consisting of (a1) a glycylglycine derivative represented by formula (1) or a salt thereof, (a2) a polyglycerin having an average degree of polymerization of 2 or more and 6 or less, and (a3) an alkyl glyceryl ether having an alkyl group having 6 or more and 40 or less carbon atoms.

### <Component (a1): a glycylglycine derivative represented by formula (1) or a salt thereof>

Component (a1) is a glycylglycine derivative represented by formula (1) or a salt thereof. 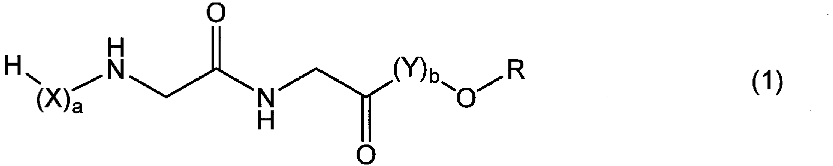
wherein X represents a divalent hydrocarbon group having 1 to 4 carbon atoms and optionally substituted with hydroxyl group(s), or an amino acid residue,
Y represents an amino acid residue or a divalent group represented by formula (2) 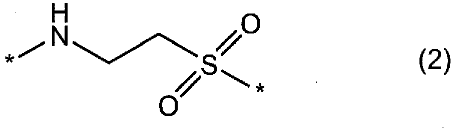
wherein -* represents a bond to an adjacent carbonyl group or an oxygen atom,
R represents a hydrogen atom or a monovalent hydrocarbon group having 1 to 4 carbon atoms and optionally substituted with hydroxyl group(s), and
a and b represent 0 or 1, with the proviso that if a and b simultaneously represent 1, then X does not represent an amino acid residue.

The glycylglycine derivative represented by formula (1) above or a salt thereof may be present as a free compound or an amphoteric ion.

Examples of the salt of the glycylglycine derivative include an inorganic acid salt such as a hydrochloride and a sulfate; an organic acid salt such as a lactate; an ammonium salt (NH⁴⁺); an organic ammonium salt such as an alkyl ammonium salt; and an alkali metal salt such as a sodium salt.

In formula (1), a divalent hydrocarbon group having 1 to 4 carbon atoms, optionally substituted with hydroxyl group(s) and represented by X may be saturated or unsaturated, or a linear or branched. In particular, a divalent saturated hydrocarbon group substituted with hydroxyl group(s) or a divalent saturated hydrocarbon group is preferable in order to improve durability of a wavy/curly hair relaxation effect against shampooing.

Examples of the divalent hydrocarbon group include a methylene group, an ethylene group, an ethylidene group, a vinylene group, a trimethylene group, an isopropylidene group, a 1-propenylene group, a tetramethylene group, a 2-methyltrimethylene group, a 1-methyltrimethylene group, and a 1-butenylene group.

Examples of the divalent hydrocarbon group substituted with hydroxyl group(s) include a 1-hydroxyethylene group, a 1-hydroxytrimethylene group, a 1,2-dihydroxytrimethylene group, a 1-hydroxytetramethylene group, a 1,2-dihydroxytetramethylene group, a 1,3-dihydroxytetramethylene group, and a 1,2,3-trihydroxytetramethylene group.

In formula (1), the "amino acid residue" represented by X refers to a unit amino acid portion derived from all amino acids synthetically obtained or present in a living body and to be used for forming an oligopeptide, and may be D-form and L-form.

Examples of the amino acid residue represented by X include a basic amino acid residue such as an arginine residue, a lysine residue, and a histidine residue; an aliphatic amino acid residue such as an alanine residue and a glycine residue; an aromatic amino acid residue such as a phenylalanine residue, a tyrosine residue, and a tryptophan residue; an acid amide amino acid residue such as a glutamine residue and an asparagine residue; an acidic amino acid residue such as a glutamate residue, an aspartic acid residue, and a cysteic acid residue; a hydroxy amino acid residue such as a serine residue and a threonine residue; and a cyclic amino acid residue such as a proline residue, an N-methylproline residue, and a 4-hydroxyproline residue. Of them, at least one selected from the group consisting of an arginine residue, an alanine residue, a phenylalanine residue, a glycine residue, a glutamine residue, a glutamate residue, a serine residue, a proline residue, an N-methylproline residue, and a 4-hydroxyproline residue is preferable, in order to improve durability of the wavy/curly hair relaxation effect against shampooing

In formula (1), examples of the amino acid residue represented by Y include the same amino acid residues as those represented by X. In order to improve durability of the wavy/curly hair relaxation effect against shampooing, at least one selected from the group consisting of an arginine residue, a alanine residue, a glycine residue, a glutamine residue, a glutamate residue, a serine residue, a proline residue, a 4-hydroxyproline residue, and a divalent group represented by chemical formula (2) above is preferable as Y.

In formula (1), a monovalent hydrocarbon group having 1 to 4 carbon atoms, optionally substituted with hydroxyl group(s) and represented by R may be a saturated or unsaturated, or linear or branched. As the monovalent hydrocarbon group, an alkyl group is preferable. Examples thereof include a methyl group, an ethyl group, a propyl group, a butyl group, an isopropyl group, an isobutyl group, an s-butyl group, and a t-butyl group.

As a monovalent hydrocarbon group substituted with hydroxyl group(s), a hydroxyalkyl group is preferable. Examples thereof include a hydroxymethyl group, a 2-hydroxyethyl group, a 3-hydroxypropyl group, a 4-hydroxybutyl group, a 2,3-dihydroxyethyl group, a 2,3,4-trihydroxybutyl group, and a 2,4-dihydroxybutyl group.

In the present invention, as a suitable example of the glycylglycine derivative, a compound represented by any one of the formulas (G1) to (G10) can be mentioned. In order to improve durability of the wavy/curly hair relaxation effect against shampooing, a compound represented by any one of the formulas (G3) to (G10) is more preferable and a compound (glycylglycylglycine or glycylglycine) represented by either one of formula (G9) and (G10) is further preferable. These glycylglycine derivatives each may be a free compound or an amphoteric ion or forms a salt. These may be used alone or in combination (two or more).

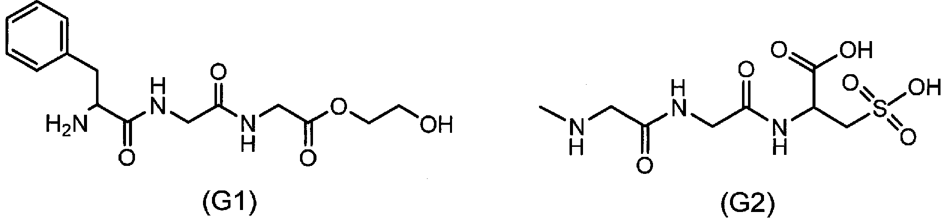

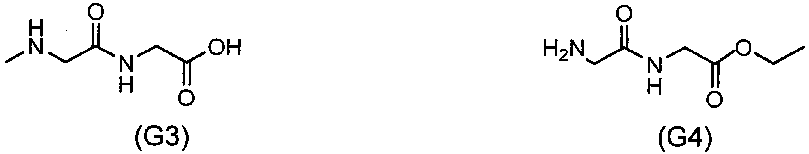

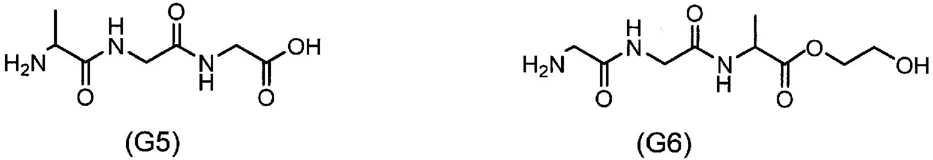

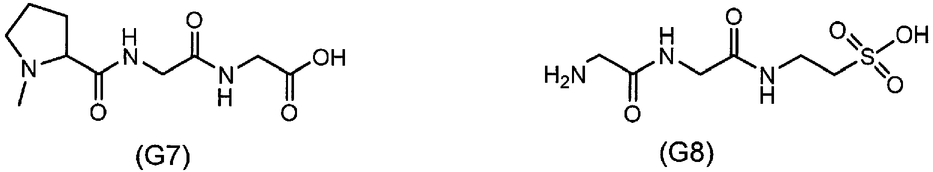

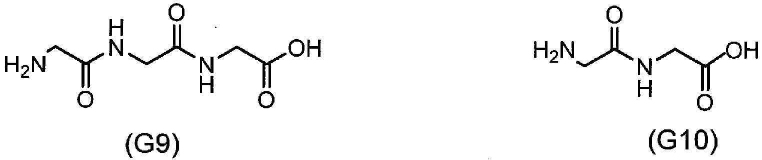

### <Component (a2): Polyglycerin of an average degree of polymerization of 2 or more and 6 or less>

As the polyglycerin of an average degree of polymerization of 2 or more and 6 or less serving as component (a2), a diglycerin, a triglycerin, a tetraglycerin, and a pentaglycerin are mentioned. In order for the component to sufficiently penetrate into the hair, the average degree of polymerization is preferably 2 or more and 4 or less and more preferably 2.5 or more and 3.5 or less.

### <Component (a3): Alkyl glyceryl ether having an alkyl group having 6 or more and 40 or less carbon atoms >

The alkyl glyceryl ether having an alkyl group having 6 or more and 40 or less carbon atoms serving as component (a3) preferably has an alkyl group having 8 or more and further 14 or more, and 20 or less and further 18 or less carbon atoms, in order to improve durability of the wavy/curly hair relaxation effect against shampooing. Also, the alkyl group is preferably a branched alkyl group. Specific examples thereof include isostearyl glyceryl ether, isodecyl glyceryl ether, and ethyl hexyl glyceryl ether.

As component (A), any one of the components (a1), (a2), and (a3) is used alone or two types or more of them are used in combination. Of them, in order to improve the wavy/curly hair relaxation effect and durability of the wavy/curly hair relaxation effect against shampooing, component (a1) is preferably contained as component (A).

In the composition containing component (A), the content of component (A) is preferably 0.5 mass% or more, more preferably 0.8 mass% or more, and further preferably 1.0 mass% or more, in order to obtain a higher durability to shampooing, whereas, the content of component (A) is preferably 10 mass% or less, more preferably 8 mass% or less, and further preferably 6 mass% or less in order to obtain not only the wavy/curly hair relaxation effect but also softer texture after hair treatment.

Further, to obtain the higher wavy/curly hair relaxation effect, durability of the wavy/curly hair relaxation effect against shampooing, and improve less detangling of treated hair, the content of component (A) in the composition is preferably 1.0 mass% or more, more preferably 1.5 mass% or more, and further preferably 2.0 mass% or more; and in order to attain not only the wavy/curly hair relaxation effect but also softer texture after hair treatment, the content of component (A) in the composition is preferably 10 mass% or less, more preferably 8 mass% or less, and further preferably 6 mass% or less.

### [Component (B): Carboxylic acid having an inorganic value and an organic value within a predetermined range or a salt of the carboxylic acid]

Component (B) is a carboxylic acid having an inorganic value of 250 or more and 450 or less and an organic value of 50 or more and 250 or less, or a salt of the carboxylic acid. Note that, the inorganic value and organic value in component (B) refers to an inorganic value and organic value of a carboxylic acid. More specifically, in the case of a salt of a carboxylic acid, the inorganic value and organic value of a free carboxylic acid are referred to. In view of the magnitude of curly-hair relaxation effect, softer texture of the treated hair, and less-detangling of hair, the inorganic value is preferably 260 or more and more preferably 265 or more; and preferably 420 or less and more preferably 400 or less. From the same points of view, the organic value is preferably 60 or more and more preferably 80 or more; and preferably 200 or less and more preferably 180 or less.

Note that, the inorganic value and organic value are values obtained in accordance with the organic conceptual diagram, and a value obtained by calculating in accordance with "Emulsification Formulation design by the organic conceptual diagram" (Yamori; Fragrance Journal, 1989 (4), P29 to 38) is used.

Such a carboxylic acid is selected from the group consisting of a carboxylic acid represented by formula (B-1), malic acid, succinic acid, and lactic acid. 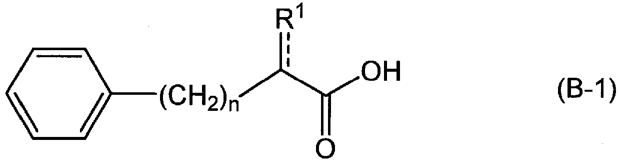 wherein R¹ represents a hydrogen atom, an oxygen atom, or hydroxyl group(s); the dashed line indicates a double bond if R¹ is an oxygen atom; n represents an integer from 0 or more and 3 or less. The phenyl group and methylene chain are optionally partly substituted with hydroxyl group(s).

As the carboxylic acid represented by formula (B-1), e.g., mandelic acid and phenyllactic acid are mentioned.

The inorganic value and organic value of these carboxylic acids are as follows: mandelic acid (265, 160), phenyllactic acid (265, 180), malic acid (400, 80), succinic acid (300, 80), and lactic acid (250, 60). Note that, the (two) numerical values within each of the parentheses represent an inorganic value and an organic value, respectively.

As component (B), in order to more strongly relax curly hair, at least one selected from the group consisting of mandelic acid, phenyllactic acid, malic acid, succinic acid, and salts of these is preferable. Of them, at least one selected from the group consisting of malic acid, mandelic acid, phenyllactic acid, and salts of these is preferable and at least one selected from the group consisting of malic acid, phenyllactic acid, and salts of these is more preferable. In order to provide softer feel and smoother feel to hair while relaxing curly hair, malic acid and a salt thereof are preferable. In order to enhance durability to shampooing while further relaxing curly hair, malic acid is preferable.

Examples of the salt of the carboxylic acid include a sodium salt, a potassium salt, a lithium salt, an aluminum salt, an ammonium salt (NH⁴⁺), an organic quaternary ammonium salt, and an arginine salt.

Note that, if the hair treatment agent to be used in step (i) is a one-part hair treatment agent containing components (A) and (B), at least one selected from the group consisting of mandelic acid, phenyllactic acid, malic acid, and salts of these is preferable as component (B) in order to more strongly relax curly hair and improve durability of the wavy/curly hair relaxation effect against shampooing.

These carboxylic acid or salts thereof can be used alone or in combination (two or more). The content of component (B) in the composition containing component (B) is preferably 1 mass% or more, more preferably 2.5 mass% or more, further preferably 5 mass% or more, further preferably 7.5 mass% or more, and further preferably 10 mass% or more in view of the curly-hair relaxation effect and durability of the curly-hair relaxation effect against shampooing; whereas, the content is preferably 25 mass% or less, more preferably 20 mass% or less, and further preferably 15 mass% or less, in order to reduce skin irritation.

### [Component (D): Thickener]

The composition containing component (A) and/or component (B) to be used in step (i) preferably contains further a thickener as component (D) in order to more strongly relax curly hair. Examples of the thickener include an anionic thickener, a cationic thickener, and a nonionic thickener.

Examples of the anionic thickener include polyacrylic acid (for example, Carbopol 941 and 981, manufactured by Noveon), an acrylic acid-alkyl methacrylate copolymer (for example, Carbopol ETD2020 manufactured by Noveon), a hydrolysate of a lower alkyl vinyl ether-maleic anhydride copolymer partially crosslinked with a terminal-unsaturated diene compound or monoalkyl ester thereof (Stabilize 06 and QM manufactured by ASHLAND), carrageenan (for example, Soageena LX22 and ML210 manufactured by Mitsubishi Rayon Co., Ltd.), xanthan gum (for example, ECHO GUM T manufactured by Dainippon Sumitomo Pharmaceutical Co., Ltd.), Welan gum (for example, K1C376 and K1A96 manufactured by Sansho Co., Ltd), hydroxypropyl xanthan gum (for example, Rhaball gum EX manufactured by Dainippon Sumitomo Pharmaceutical Co., Ltd.), sodium stearoxy PG-hydroxyethyl cellulose sulfonate and a hydroxyethyl acrylate-acryloyl dimethyl taurine Na copolymer (for example, SIMULGEL NS and SEPINOV EMT10 manufactured by SEPPIC).

Examples of the cationic thickener include a natural or semi-synthetic cationic polysaccharide and synthetic polymers containing an amino group or an ammonium group at a side chain thereof or a diallyl quaternary ammonium salt as a constitutional unit.

Examples of the cationic polysaccharide include a cationic cellulose derivative (for example, Leogard G and GP manufactured by Lion Corporation; UCARE Polymer JR-125, JR-400, JR-30M, LR-400, and LR-30M manufactured by Dow Chemical, Celquat H-100 and L-200 manufactured by Akzo Nobel), cationized guar gum derivatives (for example, Jaguar C-13S and C-17 manufactured by Solvay Advanced polymers K.K.: Rhaball gum CG-M, CG-M7, CG-M8M manufactured by DSP Gokyo Food & Chemical company), hydroxypropyl chitosan (for example, Chitofilmer HV-10 manufactured by Ichimaru Falcos), and chitosan·dl-pyrrolidone carboxylate salt(for example, Kytamer PC manufactured by Union Carbide Corporation).

As the synthetic cationic polymer containing an amino group or an ammonium group at the side chain thereof, a synthetic cationic polymer containing trialkylaminoalkyl (meth)acrylate, trialkylaminoalkyl (meth)acrylamide, (meth)acrylamide, or vinylamine as a constitutional unit is mentioned. Specific examples thereof include a polymer of methacryloyloxyethylene trimonium chloride (INCI name: polyquaternium-37; for example Cosmedia Ultra Gel 300, and SALCARE SC95 manufactured by BASF, and Synthalen CR manufactured by Sigma 3V); an (acrylic acid/methyl acrylate/3-methacryloylaminopropyltrimethylammonium chloride) copolymer (INCI name: polyquaternium-47, for example, Merquat 2201 manufactured by Lubrizol Corporation), an (acrylic acid/acrylamide/methylmethacrylamidopropyltrimethylammonium chloride) copolymer (INCI name: polyquaternium-53, for example, Merquat 2003 manufactured by Lubrizol Corporation), a (dimethyl acrylamide/ethylmethacrylatetrimonium chloride) copolymer (for example, Tinobis CD manufactured by BASF), and a (vinylamine/vinyl alcohol) copolymer (for example, SEVOL ULTALUX AD manufactured by Sekisui Specialty Chemical Company; and Diafix C-601 manufactured by Mitsubishi Chemical Corporation).

Specific examples of the synthetic cationic polymer containing a diallyl quaternary ammonium salt as a constitutional unit include a polymer of diallyldimethylammonium chloride (INCI name: polyquaternium-6, for example, Merquat 100 manufactured by Lubrizol Corporation), a (dimethyldiallylammonium chloride/acrylamide) copolymer (INCI name: polyquaternium-7, for example, Merquat 550 and 740 manufactured by Lubrizol Corporation), an (acrylic acid/diallyldimethylammonium chloride) copolymer (INCI name: polyquaternium-22, for example, Merquat 280 and 295 manufactured by Lubrizol Corporation), and an (acrylamide/acrylic acid/diallyldimethylammonium chloride) copolymer (INCI name: polyquaternium-39, for example, Merquat plus 3330 and 3331 manufactured by Lubrizol Corporation).

As specific examples of a nonionic thickening polymer, a natural or semi-synthetic nonionic thickening polysaccharide and a synthetic nonionic polymer containing e.g., vinyl alcohol or an oxyalkylene as a constitutional unit are mentioned.

As the natural or semi-synthetic nonionic polysaccharide, a water soluble natural polysaccharide such as starch, guar gum, locust bean gum, and glucomannan; and a water soluble hydroxyalkylated polysaccharide obtained by reacting an alkylene oxide with, e.g., cellulose, starch, guar gum, or locust bean gum, are mentioned. Specific examples thereof include guar gum (for example, FIBERON S manufactured by DSP Gokyo Food & Chemical Co. Ltd.), pullulan (for example, Pullulan PI-20 manufactured by Hayashibara Co., Ltd.), hydroxyethyl cellulose (for example, SE-850 manufactured by Daicel FineChem Ltd. and CELLOSIZE HEC QP-52000-H manufactured by Dow Chemical), methyl hydroxyethyl cellulose (STRUCTURE CELL 12000M manufactured by Akzo Nobel), hydroxypropyl cellulose (for example, HPC-H, HPC-M, and HPC-L manufactured by Nippon Soda Co., Ltd.), and hydroxypropyl methylcellulose (for example, METOLOSE 60SH-10000 manufactured by Shin-Etsu Chemical Co., Ltd.) .

Specific examples of the synthetic nonionic thickening polymer containing vinyl alcohol or an oxyalkylene as a constitutional unit include a polyvinyl alcohol (for example, GOHSENOL EG-40, GH-05, KH-20, and NH-26 manufactured by Nippon Synthetic Chemical Industry Co., Ltd.), a highly polymerized polyethylene glycol (for example, POLYOX WSR N-60K, WSR301, and WSR303 manufactured by Dow Chemical and a (PEG-240/decyl tetradeceth-20/HDI) copolymer (for example, ADEKANOL GT-700 manufactured by ADEKA CORPORATION).

Of these thickeners, a natural or semi-synthetic polysaccharide is preferable in order to suppress the water evaporation rate after application, accelerate penetration of component (A) and/or component (B) into the hair to sufficiently produce the curly-hair relaxation effect; at least one selected from the group consisting of xanthan gum, hydroxy xanthan gum, and hydroxyethyl cellulose is more preferable; and at least one selected from the group consisting of xanthan gum and hydroxy xanthan gum is more preferable.

The thickeners can be used alone or in combination (two or more). The content of component (D) in a composition containing component (A) and/or component (B) is preferably 0.1 mass% or more, more preferably 0.5 mass% or more, and further preferably 1 mass% or more; and preferably 5 mass% or less and more preferably 3 mass% or less.

### [Solvent]

A composition containing component (A) preferably contains water as a solvent in order for component (A) to sufficiently penetrate into the hair. A composition containing component (B) preferably contains water as a solvent in order to improve durability of the wavy/curly hair relaxation effect against shampooing.

### [Conditions of the composition containing component (B)]

A composition containing component (B) does not contain an aromatic sulfonic acid or a salt thereof, which is component (C) contained in a hair treatment agent and used in step (iii) described later; in other words, the content thereof is preferably 0 mass%. Even if the composition contains component (C) (for example, the content thereof in the composition is 0.0001 mass% or more), the content of component (C) in the composition is preferably less than 3 mass%, more preferably less than 1 mass%, and further preferably less than 0.5 mass%.

The composition containing component (B) can contain an aromatic alcohol as component (E) later described. Not only to sufficiently produce the curly-hair relaxation effect but also to obtain the composition as a uniform preparation, the content of component (E) is preferably low as long as it can be dissolved in the composition even if the content of component (F) (described later) is low, and more preferably component (E) is not contained; in other words, the content thereof is 0 mass%. If the composition contains component (E) (for example, 0.0001 mass% or more in the composition), the content of component (E) in the composition is preferably less than 5 mass%, more preferably less than 2 mass%, and further preferably less than 1 mass%.

The composition containing component (B) may further contain an organic solvent as component (F) later described. In order to sufficiently produce the curly-hair relaxation effect, the content of component (F) in the composition is preferably low and more preferably component (F) is not contained (in other words, the content is 0 mass%). If the composition contains component (F) (for example, 0.0001 mass% or more in the composition), the content of component (F) in the composition is preferably less than 5 mass%, more preferably less than 2 mass%, further preferably less than 1 mass%, and further preferably less than 0.5 mass%.

The composition containing component (B) can further contain a surfactant. However, not only to sufficiently produce the curly-hair relaxation effect but also to obtain the composition as a uniform preparation, the content of a surfactant in the composition is preferably 2 mass% or less, more preferably 1 mass% or less, further preferably 0.1 mass% or less, further preferably 0.01 mass% or less, and further preferably 0 mass%.

### [Conditions of the composition containing component (A)]

The composition containing component (A) may contain components other than components (A) and (D) and a solvent, and the components are not particularly limited. If the above composition is the one-part hair treatment agent containing components (A) and (B), it is preferable that the same conditions of the components (C), (E), and (F) and surfactant in the composition containing component (B) shall apply to the composition containing component (A). It is preferable that organic acid is used for adjusting pH of the above composition. The organic acid for use in pH adjustment is not particularly limited.

### [pH]

The pH of a composition containing component (A) (excluding the composition containing component (B)) is preferably 7.5 or less and more preferably 7.0 or less in order to accelerate penetration of component (C), i.e., an aromatic sulfonic acid or a salt thereof, into the hair in step (iii) and further preferably 6.5 or less and further preferably 6.0 or less in order to further relax curly hair. The pH of the composition is preferably 2 or more, more preferably 2.5 or more, and further preferably 2.8 or more in order to in order to accelerate penetration of component (C), i.e., an aromatic sulfonic acid or a salt thereof, into the hair in step (iii) and further relax curly hair. Note that, the pH of the composition is specified as a value measured by a pH meter at a temperature of 25°C.

The pH of a composition containing component (B), (excluding the composition containing component (A)), which may fall within any range as long as the composition can be generally used as a hair cosmetic, is preferably 2 or more, more preferably 3 or more, further preferably 3.5 or more, further preferably 4 or more, and further preferably 5 or more; and preferably 10 or less, more preferably 9 or less, and further preferably 8 or less. In order to sufficiently produce the curly-hair relaxation effect, further reduce damage to hair and the scalp, improve the feel when the composition is applied to hair and easily apply the composition to hair, the pH of the composition more preferably falls, in particular, within the range of beyond 5 and further preferably with the range of 6 or more. In contrast, to further relax curly hair, the composition is preferably acidic. If priority is given to the curly-hair relaxation effect, the pH of the composition is preferably 7 or less and more preferably 5 or less; and preferably 3 or more and more preferably 4 or more. Note that, the pH of the composition is specified as a value measured by a pH meter at a temperature of 25°C.

The pH of the composition containing components (A) and (B) is preferably 2 or more, more preferably 2.5 or more, and further preferably 2.8 or more, in order to accelerate penetration of component (C), i.e., an aromatic sulfonic acid or a salt thereof, into the hair in step (iii) and to further relax curly hair; and preferably 10 or less, more preferably 9 or less, and further preferably 8 or less. In order to sufficiently produce the curly-hair relaxation effect, further reduce damage to hair and the scalp, improve the composition texture when the composition is applied to hair and easily apply the composition to hair, the pH of the composition more preferably falls, in particular, within the range of beyond 5 and further preferably 6 or more. In contrast, to further relax curly hair, the composition is preferably acidic. If priority is given to the curly-hair relaxation effect, the pH of the composition is preferably 7.5 or less, more preferably 7.0 or less, further preferably 6.5 or less, and further preferably 6.0 or less; and preferably 3 or more and more preferably 4 or more. Note that, the pH of the composition is specified as a value measured by a pH meter at a temperature of 25°C.

### • Hair treatment agent to be used in step (iii)

The hair treatment agent to be used in step (iii) primarily contains an active ingredient to penetrate the hair.

### [Component (C): Aromatic sulfonic acid having a molecular weight of 300 or less, or a salt thereof]

As the aromatic sulfonic acid having a molecular weight of 300 or less, or a salt thereof contained as component (C) in the hair treatment agent to be used in step (iii), for example naphthalene sulfonic acid, azulene sulfonic acid, benzophenone sulfonic acid, and benzene sulfonic acid, are mentioned.

Examples of the naphthalene sulfonic acid include compounds represented by the following formula (3). 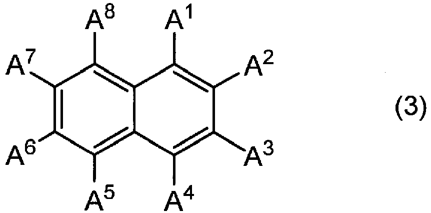 wherein one or more of A¹ to A⁸ represent a sulfo group or a salt thereof, the rest represent a hydrogen atom, a halogen atom, hydroxyl group(s), a nitro group, a carboxy group, a lower alkoxycarbonyl group, an alkyl group, an alkenyl group, a lower alkoxy group, a formyl group, an acyl group, a phenylazo group optionally having a substituent or -N(R')(R'') where R' and R" represent a hydrogen atom, a lower alkyl group, a lower alkenyl group, a phenyl group, a benzyl group, or an acyl group.

Specific examples of the naphthalene sulfonic acids include 1-or 2-naphthalene sulfonic acid (α- or β-naphthalene sulfonic acid), 2,7-naphthalenedisulfonic acid, 1,5-naphthalenedisulfonic acid, 2,6-naphthalenedisulfonic acid, 1-naphthol-2-sulfonic acid, 1-naphthol-4-sulfonic acid, 2-naphthol-6-sulfonic acid, 2-naphthol-7-sulfonic acid, 2,3-dihydroxynaphthalene-6-sulfonic acid, 1,7-dihydroxynaphthalene-3-sulfonic acid, chromotropic acid (4,5-dihydroxynaphthalene-2,7-disulfonic acid), 3,6-dihydroxynaphthalene-2,7-disulfonic acid, S-acid (1-amino-8-naphthol-4-sulfonic acid), gamma acid (2-amino-8-naphthol-6-sulfonic acid), J-acid (2-amino-5-naphthol-7-sulfonic acid), 1-amino-2-naphthol-4-sulfonic acid, 1-naphthylamine-4-sulfonic acid, Bronner's acid (2-naphthylamine-6-sulfonic acid), Cleve's acid (1-naphthylamine-7-sulfonic acid), 2-naphthylamine-1-sulfonic acid, 1-naphthylamine-6-sulfonic acid, 1-naphthylamine-8-sulfonic acid, 2,7-diamino-1-naphthol-3-sulfonic acid, 7,8-diamino-1-naphthol-3-sulfonic acid, a naphthalene sulfonic acid formalin polycondensate having a molecular weight of 300 or less, 6-methyl-2-naphthalene sulfonic acid, 4-ethyl-1-naphthalene sulfonic acid, 5-isopropyl-1-naphthalene sulfonic acid, 5-butyl-2-naphthalene sulfonic acid, and salts of these.

Specific examples of the azulene sulfonic acid include guaiazulenesulfonic acid, 1-azulenesulfonic acid, 3-acetyl-7-isopropyl-1-azulenesulfonic acid, 3-(2-hydroxyethyl)-7-isopropyl-1-azulenesulfonic acid, 3-methyl-7-isopropyl-1-azulenesulfonic acid, 7-isopropyl-1-azulenesulfonic acid, 3-phenyl-6-isopropyl-1-azulenesulfonic acid, 1,4-dimethyl-7-isopropyl-2-azulenesulfonic acid, 4-ethoxy-3-ethyl-6-isopropyl-1-azulenesulfonic acid, 1,3-azulenedisulfonic acid, 4,6,8-trimethyl-1,3-azulenedisulfonic acid, 3-formyl-4,6,8-trimethyl-1-azulenesulfonic acid, and salts of these.

As the benzophenone sulfonic acid, for example, compounds represented by the following formula (4) are mentioned. 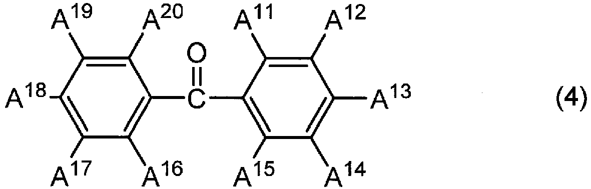 wherein one or more of A¹¹ to A²⁰ represent a sulfo group or a salt thereof, the rest represent a hydrogen atom, a halogen atom, hydroxyl group(s), a carboxy group, an amino group, a lower alkyl group, a lower alkenyl group, a lower alkoxy group, or an acyl group.

Specific examples of the benzophenone sulfonic acid include oxybenzene sulfonic acid, o-chlorobenzophenone sulfonic acid, p-chlorobenzophenone sulfonic acid, 2-hydroxybenzophenone sulfonic acid, 4-hydroxybenzophenone sulfonic acid, 2-aminobenzophenone sulfonic acid, 4-aminobenzophenone sulfonic acid, 2-methylbenzophenone sulfonic acid, 4-methoxybenzophenone sulfonic acid, 4,4'-dimethylbenzophenone sulfonic acid, 4,4'-dimethoxybenzophenone sulfonic acid, and salts of these.

As the benzene sulfonic acid, for example, compounds represented by the following formula (5) are mentioned. 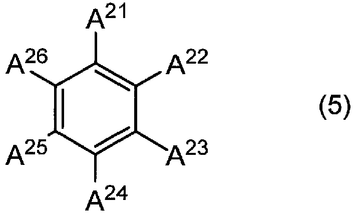 wherein one or more of A²¹ to A²⁶ represent a sulfo group or a salt thereof and the rest represent a hydrogen atom or a lower alkyl group.

Specific examples of the benzene sulfonic acid include benzenesulfonic acid, o-toluenesulfonic acid, p-toluenesulfonic acid, xylene sulfonic acid, cumene sulfonic acid, ethyl benzene sulfonic acid, 2,4,6-trimethylbenzenesulfonic acid, and salts of these.

The salts of the aromatic sulfonic acids include a sodium salt, a potassium salt, a lithium salt, an aluminum salt, an ammonium salt (NH⁴⁺), and an organic ammonium salt.

As the aromatic sulfonic acid or a salt thereof as component (C), at least one selected from the group consisting of naphthalene sulfonic acid represented by formula (3), benzophenone sulfonic acid represented by formula (4), and benzene sulfonic acid represented by formula (5) is preferable in order to further strongly relax curly hair and provide softer feel and provide less detangled hair, and further, at least one selected from 2-naphthalene sulfonic acid (β-naphthalene sulfonic acid), 1-naphthalene sulfonic acid (α-naphthalene sulfonic acid), p-toluenesulfonic acid, xylene sulfonic acid, hydroxymethoxy benzophenone sulfonic acid (oxybenzone-5), and salts of these is more preferable. Of them, from the aforementioned point of view, 1-or 2-naphthalene sulfonic acid (α-or β-naphthalene sulfonic acid) or a salt thereof is further preferable.

These aromatic sulfonic acids or salts thereof can be used alone or in combination (two or more). The content of component (C) in the hair treatment agent to be used in step (iii) is preferably 1 mass% or more, more preferably 2.5 mass% or more, further preferably 4 mass% or more, further preferably 5 mass% or more, and further preferably 7 mass% or more in order to improve the curly-hair relaxation effect, durability of the curly-hair relaxation effect against shampooing, and the smoothness of the treated hair. In order to improve smoothness of the treated hair and less detangling of hair, the content is preferably 25 mass% or less, more preferably 20 mass% or less, further preferably 15 mass% or less, and further preferably 10 mass% or less.

### [Component (D): Thickener]

The hair treatment agent to be used in step (iii) preferably contains further the aforementioned thickener as component (D) in order to more strongly relax curly hair. The thickeners can be used alone or in combination (two or more). The content of component (D) in the hair treatment agent to be used in step (iii) is preferably 0.1 mass% or more, more preferably 0.5 mass% or more, and further preferably 1.0 mass% or more; and preferably 5 mass% or less and more preferably 3 mass% or less in order to accelerate penetration of component (C) into the hair to sufficiently produce the curly-hair relaxation effect.

### [Component (E): Aromatic alcohol]

The hair treatment agent to be used in step (iii) can further contain an aromatic alcohol as component (E) in order to obtain both the strong curly-hair relaxation effect and less detangling of hair. Examples of such an aromatic alcohol include benzyl alcohol, cinnamyl alcohol, phenethyl alcohol, p-anisyl alcohol, p-methylbenzyl alcohol, phenoxyethanol, and 2-(benzyloxy)ethanol. Of these, in view of compatibility with an aromatic sulfonic acid, at least one selected from the group consisting of benzyl alcohol and 2-(benzyloxy)ethanol is preferable and benzyl alcohol is more preferable.

These aromatic alcohols can be used alone or in combination (two or more). In the hair treatment agent to be used in step (iii), the content of component (E) is preferably 1 mass% or more, more preferably 2 mass% or more, and further preferably 4 mass% or more in view of the wavy/curly hair relaxation effect and improvement of less detangling of hair. From the same points of view, the content is preferably 30 mass% or less, more preferably 20 mass% or less, further preferably 15 mass% or less, and further preferably 10 mass% or less.

In the hair treatment agent to be used in step (iii), the mass ratio (C)/(E) of component (C) to component (E) is preferably 0.25 or more, more preferably 0.3 or more, further preferably 0.35 or more, and further preferably 0.5 or more, in order to remarkably improve the curly-hair relaxation effect. From the same points of view, the mass ratio is preferably 2.5 or less, more preferably 2 or less, further preferably 1.8 or less, further preferably 1 or less, further preferably 0.75, and further preferably 0.7 or less.

### [Component (F): Organic solvent]

The hair treatment agent to be used in step (iii) may further contain an organic solvent represented by the following formula (F-1) as component (F).

R²-(OCH₂CH₂)_{q}-R³ (F-1)

wherein R² represents a hydrogen atom or an alkyl group having 1 or more and 5 or less carbon atoms; R³ represents a hydrogen atom or hydroxyl group(s); q represents an integer of 0 or more and 5 or less, with the proviso that if q is 0, then R² and R³ are not hydrogen atoms.

As component (F), a monovalent alcohol having 1 or more and 4 or less carbon atoms and ethylene glycol monoalkyl ether where R² represents a linear or branched alkyl having 1 or more and 5 or less carbon atoms and q represents an integer of 1 or more and 5 or less, are mentioned. Specific examples thereof include ethanol, propanol, isopropanol, ethylene glycol monomethyl ether, ethylene glycol monoethyl ether, ethylene glycol monobutyl ether, diethylene glycol monomethyl ether, diethylene glycol monoethyl ether, and diethylene glycol monobutyl ether.

In order to sufficiently produce the curly-hair relaxation effect, the content of component (F) in the hair treatment agent to be used in step (iii) is preferably low and more preferably component (F) is not contained; in other words, the content is 0 mass%. If the content of component (F) in the system is large, the curly-hair relaxation effect, which is improved by interaction between component (C), i.e., an aromatic sulfonic acid, and component (E), i.e., an aromatic alcohol, may be undermined. The content of component (F) is preferably suppressed within the concentration range where the effect is not undermined. More specifically, component (F) can be contained such that the mass ratio of component (F) relative to the total of component (C) and component (E), i.e., (F)/[(C) + (E)], falls within the range of 0.3 or less. The mass ratio (F)/[(C) + (E)] is more preferably 0.2 or less and further preferably 0.1 or less. Further preferably, the mass ratio (F)/[(C) + (E)] is 0; in other words, component (F) is preferably not contained.

In the hair treatment agent to be used in step (iii), the content of component (F) is preferably 3 mass% or less, more preferably 2 mass% or less, further preferably 1 mass% or less, and further preferably 0 mass% from the same point of view as above.

### [Component (G): Polyhydric alcohol]

The hair treatment agent to be used in step (iii) can further contain a polyhydric alcohol as component (G). Examples of the polyhydric alcohol include polyhydric alcohols having 2 to 20 carbon atoms, including an alkylene glycol such as ethylene glycol, diethylene glycol, triethylene glycol, polyethylene glycol, propylene glycol, dipropylene glycol, polypropylene glycol, 1,2-butylene glycol, 1,3-butylene glycol, pentylene glycol, and hexylene glycol; glycerin such as glycerin and polyglycerin except that used as component (A); sugar alcohol such as xylit, mannit, garactit, and sorbit; and other alcohols such as trimethylol ethane, trimethylol propane, and pentaerythritol. Of them, in order to improve the curly-hair relaxation effect and the feel of treated hair, an alkylene glycol is preferable, more specifically, at least one selected from the group consisting of propylene glycol, dipropylene glycol, and polypropylene glycol.

These polyhydric alcohols can be used alone or in combination (two or more). In the hair treatment agent to be used in step (iii), the content of component (G) is preferably 5 mass% or more, more preferably 10 mass% or more, and further preferably 15 mass% or more; and preferably 30 mass% or less, more preferably 25 mass% or less, and further preferably 20 mass% or less.

### [Polymeric surfactant]

The hair treatment agent to be used in step (iii) can further contain a polymeric surfactant in order to remarkably improve the curly-hair relaxation effect. As the polymeric surfactant, a silicone surfactant such as an oxazoline modified silicone (e.g., polysilicone-9), a polyether modified silicone, and an aminopolyether modified silicone, are preferable.

### [Solvent]

The hair treatment agent to be used in step (iii) preferably contains water as a solvent in order for component (C) to sufficiently penetrate into the hair.

### [pH]

The pH of the hair treatment agent to be used in step (iii) may fall within any range as long as the hair treatment agent is generally used as a hair cosmetic. In order not to damage hair and the scalp, pH is preferably 3 or more and 10 or less and more preferably 3.5 or more and 8 or less. To sufficiently produce the curly-hair relaxation effect, to improve the feel when the hair treatment agent to be used in step (iii) is applied to the hair, and to easily apply the composition to the hair, the pH of the hair treatment agent to be used in step (iii) is more preferably 5 or more and 8 or less. Note that, the pH of the hair treatment agent to be used in step (iii) is specified as a value obtained by measuring the pH of the hair treatment agent diluted 10 fold with deionized water by a pH meter at a temperature of 25°C.

The hair treatment agents to be used in steps (i) and (iii) preferably do not substantially contain further a hair reducing agent in order to suppress chemical damage to hair. The present invention is characterized in that hair can be deformed without cleaving an S-S bond of a protein in the hair and employs a technique totally different from that employed in a perm chemical, which deforms hair by cleaving an S-S bond of a protein in the hair by a reducing agent. As the hair reducing agent, a thiol such as thioglycolic acid, dithioglycolic acid, cysteine, and acetylcysteine and hydrogen sulfite and a salt thereof, are mentioned.

Note that, in the present invention, "not substantially contained" means that the content of a target compound in the hair treatment agent is preferably less than 0.1 mass%, more preferably less than 0.01 mass%, and further preferably means that no target compound is contained in the hair treatment agent.

### • Hair treatment method

The hair treatment of the present invention can be carried out by the hair treatment method having the following steps (i) to (iii) .
step (i): a step of applying the hair treatment agent containing components (A) and (B) to hair;
step (ii): a step of leaving the hair at 15°C or more and 100°C or less for 15 seconds or more and 60 minutes or less, after step (i) ;
step (iii): a step of applying the hair treatment agent containing component (C) to the hair, after step (ii),
wherein the method does not comprise applying a hair treatment agent containing a reducing agent.

Before step (i), hair is preferably detangled by hand or using a tool such as a comb and a brush in order to prevent hair damage. Although hair may be shampooed or not before step (i), if a step of shampooing the hair is included, e.g., a commercially available shampoo can be used for washing.

### [Step (i): Step of applying the hair treatment agent containing components (A) and (B) to hair]

Step (i) is a step of applying the hair treatment agent containing components (A) and (B) to hair. Owing to component (B) in addition to component (A) contained in the hair treatment agent, curly hair can be more relaxed; the relaxed curly-hair strands flows more evenly; the curly hair is more manageable; the shape just after the hair treatment can be maintained even if the hair is repeatedly shampooed; and further, the treated hair can be softer, naturally smooth, and less detangled.

Specific procedures of step (i), which vary depending on the type of the hair treatment agent, will be separately described below. In the following description on the hair treatment method, the simple description "hair treatment agent" refers to a composition actually applied to hair and collectively refers to a one-part hair treatment agent; a mixture of a first part and a second part of a single-application type multi-part hair treatment agent; and each of the first part and the second part of a sequential-application type multi-part hair treatment agent.

### • One-part hair treatment agent

Step (i-a): a step of applying the one-part hair treatment agent to hair

### • Single-application multi-part hair treatment agent

Step (i-b): a step of applying a mixture of the first part and the second part of the multi-part hair treatment agent

### • Sequential-application multi-part hair treatment agent

Step (i-c): a step of applying the first part of the multi-part hair treatment agent and thereafter applying the second part thereof over the first part application portion of the hair.

### Remarks common to step (i-a), step (i-b), and step (i-c)

In any one of the above steps, the hair treatment agent may be applied to dry hair or wet hair; however, the hair treatment agent is preferably applied to the dry hair. A target hair for treatment may be the whole or part of head hair.

In step (i), the amount of the hair treatment agent to be applied to hair, i.e., a liquor ratio to the mass of hair (mass of the hair treatment agent/mass of hair) is preferably 0.05 or more, more preferably 0.1 or more, and further preferably 0.25 or more; and preferably 1.5 or less, more preferably 1.25 or less, and further preferably 1.0 or less.

The hair treatment agent is applied to hair and thereafter spread into the whole hair by massaging the composition (agent) into the hair and/or combing hair by hand or a tool such as a brush, a comb, and a hair brush, or by combination of both of them.

### Step (i-a) and step (i-b)

If the one-part hair treatment agent or the single-application type multi-part hair treatment agent is used, more specifically, in step (i-a) or step (i-b), component (A) and component (B) are simultaneously applied to hair. In this case, it is not necessary to provide interval between applications of both components. Because of this, these embodiments are preferable in order to reduce treatment time.

### Step (i-c)

If the sequential-application type multi-part hair treatment agent is used, the first part is applied to hair in accordance with the aforementioned conditions, and thereafter the second part may be applied to the hair in accordance with the aforementioned conditions. Herein, it is preferable that the second part is applied to the portion to which the first part is already applied. It is acceptable that one of the first part and the second part contains component (A) and the other contains component (B). More specifically, the first part containing component (A) is applied to hair, and thereafter the second part containing component (B) may be applied to the portion to which the first part is already applied so as to overlap it; or alternatively the first part containing component (B) is applied to hair, and thereafter the second part containing component (A) is applied to the portion to which the first part is already applied so as to overlap it.

If a sequential-application type multi-part hair treatment agent is used, after the first part is applied to the hair and before the second part is applied to the hair, the following step (i-cx) is preferably carried out.
step (i-cx): a step of leaving the hair to stand at 15°C or more and 100°C or less for 15 seconds or more and 60 minutes or less.

The temperature of step (i-cx) of leaving the hair to stand is 15°C or more and 100°C or less, preferably 60°C or less, and more preferably 30°C or less. The temperature is preferably 15°C or more and less than 30°C and more specifically room temperature in order to simply treat hair without using a special apparatus. In contrast, in order to reduce the time of standing, the hair may be left to stand while warming by, e.g., a heater. In this case, the temperature is preferably 30°C or more and more preferably 40°C or more; and preferably 100°C or less and more preferably 60°C or less.

The time of step (i-cx) of leaving the hair to stand is 15 seconds or more in order to relax curly hair, provide softer feel to the hair, and allow the treated hair to be less entangled, preferably 30 seconds or more, more preferably 1 minute or more, preferably 3 minutes or more, and more preferably 5 minutes or more; and 60 minutes or less, preferably 45 minutes or less, and more preferably 30 minutes or less. If the hair is left to stand while heating by, e.g., a heater as mentioned above, the time of standing can be further reduced. In this case, the time of leaving the hair to stand is 15 seconds or more, preferably 30 seconds or more, more preferably 1 minute or more, preferably 3 minutes or more, and more preferably 5 minutes or more; and preferably 30 minutes or less, more preferably 20 minutes or less, and further preferably 15 minutes or less.

In the present invention, the time of step (i-cx) of leaving the hair to stand is specified as the time after the first part is applied to hair until the next step except the step of leaving the hair to stand.

Generally, it is advantageous to allow the hair to stand for a long time while heating in order for a component to penetrate into the hair; however, the present invention is excellent in that the effect of the invention can be obtained even if the hair is left to stand at room temperature even for a short time.

Step (i-cx) is preferably carried out under a condition where water vaporization is suppressed. As a specific means for suppressing the water vaporization, a means for covering the hair, to which the first part is applied, for example, with a plastic film or cap, and a means for continuously spraying water vapor such as superheated steam to hair, are mentioned.

After the hair is left to stand and before the second part is applied, the first part is rinsed out or not from the hair. If the hair is rinsed after standing, the rinsed hair may be dried or not before the second part is applied. The rinsed hair may be detangled or not, by hand or using a tool such as a comb and a brush before another composition is applied.

After the hair is left to stand in step (i-cx), the second part may be applied to the hair in accordance with the aforementioned conditions.

### [Step (ii): Step of leaving the hair to stand at 15°C or more and 100°C or less for 15 seconds or more and 60 minutes or less, after step (i)]

The temperature of step (ii) of leaving the hair to stand is 15°C or more and 100°C or less, preferably 60°C or less, and more preferably 30°C or less. In order to simply treat hair without using a special apparatus, the temperature is preferably 15°C or more and less than 30°C, more specifically, room temperature. In contrast, to reduce the time of leaving the hair to stand, the hair may be left to stand while warming by, e.g., a heater. In this case, the temperature is preferably 30°C or more and more preferably 40°C or more; and 100°C or less and more preferably 60°C or less.

The time of step (ii) of leaving the hair to stand is 15 seconds or more, preferably 30 seconds or more, more preferably 1 minute or more, preferably 3 minutes or more, and more preferably 5 minutes or more in order to relax curly hair and for the treated hair to have softer feel and to be less detangled; and 60 minutes or less, preferably 45 minutes or less, and more preferably 30 minutes or less. If the hair is left to stand while heating with, e.g., a heater, as mentioned above, the time of leaving the hair to stand can be further reduced. In this case, the time of standing is 15 seconds or more, preferably 30 seconds or more, more preferably 1 minute or more, preferably 3 minutes or more, and more preferably 5 minutes or more; and preferably 30 minutes or less, more preferably 15 minutes or less, and further preferably 10 minutes or less.

In the present invention, the time of leaving the hair to stand in step (ii) means the time after a second part is applied to the hair to the next step except a step of leaving the hair to stand.

Generally, it is advantageous to allow the hair to stand for a long time while heating in order for a component to penetrate into the hair; however, the present invention is excellent in that the effect of the invention can be obtained even if the hair is left to stand at room temperature even for a short time.

Step (ii) is preferably carried out under a condition where water vaporization is suppressed. As a specific means for suppressing water vaporization, a means for covering the hair, to which the hair treatment agent containing components (A) and (B) is applied, for example, with a plastic film or cap, and a means for continuously spraying water vapor such as superheated steam to hair, are mentioned.

After step (ii) and before step (iii), the hair treatment agent containing components (A) and (B) may be rinsed out or not from the hair. If the hair is rinsed after step (ii) and before step (iii), the rinsed hair may be dried or not before step (iii). The rinsed hair may be detangled or not, by hand or using a tool such as a comb and a brush before step (iii). Note that, after step (ii) and before step (iii), a step of shampooing the hair may be carried out.

### [Step (iii): Step of applying the hair treatment agent containing component (C) to the hair, after step (ii)]

In step (iii), the amount of the hair treatment agent containing component (C) to be applied to hair, i.e., a liquor ratio to the mass of hair (mass of the hair treatment agent/mass of hair) is preferably 0.05 or more, more preferably 0.1 or more, and further preferably 0.2 or more, in order to improve the wavy/curly hair relaxation effect after the hair treatment; and preferably 1.5 or less, more preferably 1.25 or less, and further preferably 1.0 or less. A target hair for treatment may be the whole or part of head hair; however, the hair treatment agent containing component (C) is preferably applied to the portion to which the hair treatment agent containing components (A) and (B) is already applied in step (i).

The hair treatment agent is applied to hair, and thereafter, spread into the whole hair by massaging it into the hair and/or combing hair by hand or a tool such as a brush, a comb, and a hair brush, or by combination of both of them.

The hair treatment method of the present invention includes not only an embodiment in which step (i), step (ii), and step (iii) are carried out sequentially in this order, but also an embodiment in which two or three of step (i), step (ii), and step (iii) are simultaneously carried out.

More specifically, step (i) is applied to part of hair. This portion is left to stand in accordance with step (ii); at the same time, step (i) may be applied to a portion to which step (i) is not yet applied. Thereafter, if step (i) is further applied to a portion to which step (i) is not yet applied, step (iii) is applied to the hair portion to which application of step (ii) is completed and step (ii) is applied to the hair portion to which application of step (i) is completed. In this case, step (i), step (ii), and step (iii) are simultaneously carried out. In short, to each portion of hair, the steps are applied sequentially in the order of step (i) -> step (ii) -> step (iii).

### [Step (iv): Step of leaving the hair to stand after step (iii)]

After step (iii), the hair may be rinsed or not; however, the hair is preferably rinsed, in order to improve the smoothness of the treated hair and obtain satisfactory less detangling. The present invention may have a step of shampooing the hair after step (iii) .

If the hair is rinsed after step (iii), further step (iv) of leaving the hair, to which the hair treatment agent is applied in step (iii) to stand at 15°C or more and 100°C or less for 1 minute or more and 60 minutes or less may be preferably included between step (iii) and a step of rinsing the hair, in order to sufficiently obtain the effect of the present invention.

In step (iv), the temperature of leaving the hair to stand is 15°C or more and 100°C or less, preferably 60°C or less, and more preferably 30°C or less. The temperature is preferably 15°C or more and less than 30°C and more specifically room temperature in order to simply treat hair without using a special apparatus. In contrast, in order to reduce the time of standing, the hair may be left to stand while warming by, e.g., a heater. In this case, the temperature is preferably 30°C or more and more preferably 40°C or more; and preferably 100°C or less and more preferably 60°C or less.

The time of leaving the hair to stand in step (iv), after the hair treatment agent is applied in step (iii), is 1 minute or more, preferably 3 minutes or more, and more preferably 5 minutes or more; and, 60 minutes or less, preferably 45 minutes or less, and more preferably 30 minutes or less in order to relax wavy/curly hair, provide softer feel to the hair, allow the hair strands to flow evenly, and provide manageability. If the hair is left to stand while heating with, e.g., a heater, as mentioned above, the time of leaving the hair to stand can be further reduced. In this case, the time of standing is 1 minute or more, preferably 3 minutes or more, and more preferably 5 minutes or more; and preferably 30 minutes or less, more preferably 15 minutes or less, and further preferably 10 minutes or less.

In the present invention, the time of leaving the hair to stand in step (iv) means the time after the hair treatment agent is applied to hair in step (iii) to the next step except the step of leaving the hair to stand.

Generally, it is advantageous to allow the hair to stand for a long time while heating in order for a component to penetrate into the hair; however, the present invention is excellent in that the effect of the invention can be obtained even if the hair is left to stand at room temperature even for a short time. In the hair treatment method of the present invention, the features of hair are changed and penetration of component (C) into the hair is accelerated by applying the hair treatment agent containing components (A) and (B) to hair in advance in step (i), with the result that such an effect can be presumably obtained.

It is preferable to carry out step (iv) in a condition where water vaporization is suppressed. As a means for suppressing water vaporization, a means for covering the hair, to which the hair treatment agent containing component (C) is applied, for example, with a plastic film or cap, and a means for continuously spraying water vapor such as superheated steam to hair, are mentioned.

If a step of rinsing the hair is carried out after step (iv), a step of shampooing the hair may be included after the step. If the step of shampooing the hair is included, e.g., a commercially available shampoo can be used. After shampooing the hair, a step of treating hair with, e.g., a commercially available conditioner and a treatment, can be included.

After the step of rinsing the hair, the hair may be dried or not. In order to prevent damage to the hair, the hair is more preferably dried. Hair can be dried naturally or applying heat by, e.g., a hair dryer. In order to reduce the treatment time, drying by heating is preferable. In order to improve manageability of hair and prevent hair damage, it is preferable that hair is appropriately detangled by hand or a tool such as a comb and a brush before, during and/or after drying.

### [Step (v): Heating step]

If straight hair (style) with no spreading from the root to a tip is wanted, further step (v) of heating the hair is preferably carried out after step (iv). The heating temperature is preferably 140°C or more, more preferably 160°C or more; and preferably 230°C or less, more preferably 200°C or less, and further preferably 180°C or less. Note that, the heating temperature refers to the temperature of an apparatus for use in the heating treatment or a condition in which the heat treatment is carried out. As a warming method, methods using, e.g., a hair iron, electric heating rods, and hot curlers are mentioned. A method of obtaining straight hair by sliding the hair iron from the root toward the tip of hair is more preferable.

The hair may be rinsed or not between step (iv) and step (v); however, the hair is preferably not rinsed, in view of the straightening of curly hair and reduction of the treatment time. Hair may be dried or not between step (iv) and step (v); however, the content of moisture is preferably reduced by, e.g., a hair dryer to suppress hair damage, and thereafter, step (v) is carried out. In order to prevent hair damage, hair is preferably detangled by hand or a tool such as a comb and a brush before step (v). If treatments of step (i) to step (iv) are applied, and then, hair is shaped in step (v), even if the hair is repeatedly shampooed, the hair shape obtained in step (v) can be almost maintained.

If a step of rinsing the hair is not included between step (iv) and step (v), a step of rinsing the hair is preferably included after step (v).

In contrast, to further reduce hair damage, simply relax curly hair without using a special apparatus, and obtain a hairstyle having hair strands flowing evenly and naturally managed, step (v) is preferably not included. Even if step (v) is not included after step (iv), the hair shape is rarely changed by the following shampooing and relaxed curly hair having strands flowing evenly and natural, manageable, and soft texture can be advantageously maintained, even if repeatedly shampooed.

In the hair treatment method of the present invention, the sufficient curly-hair relaxation effect can be obtained even if elasticity is not externally applied to hair by, e.g., a comb or a brush, during the time period after the hair treatment agent containing components (A) and (B) is applied to hair in step (i) or after the hair treatment agent containing component (C) is applied to hair in step (iii) until the hair treatment agent is removed from the hair.

As described above, the hair treatment method of the present invention is excellent in obtaining the effect of the present invention, because curly hair is autonomously relaxed without forcibly deforming the hair shape by heating or applying external force, in other words, without employing any useful operation for deforming hair.

In contrast, curly hair can be further relaxed by externally applying elasticity by, e.g., a comb or a brush during the time period after the hair treatment agent containing components (A) and (B) is applied to hair in step (i) or the hair treatment agent containing component (C) is applied to hair in step (iii) until the hair treatment agent is removed from the hair.

The hair treatment method of the present invention employs a technology for enabling relaxation of a curly hair shape based on totally different principle from a perm treatment using a reducing agent and a so-called relaxer treatment using a strong alkali hair treatment agent. Because of this, a step of applying a hair treatment agent containing a reducing agent and a step of applying a hair treatment agent of pH 12 to 14 are not included. Accordingly, the hair treatment method of the present invention can deform hair without damaging hair.

Of the embodiments mentioned above, preferable embodiments of the present invention will be further described below.

<1> A hair treatment method comprising the following steps (i) to (iii):
   step (i): a step of applying a hair treatment agent containing the following components (A) and (B) to hair:
      (A) at least one compound selected from the group consisting of a glycylglycine derivative represented by formula (1) or a salt thereof, a polyglycerin having an average degree of polymerization of 2 or more and 6 or less, and an alkyl glyceryl ether having an alkyl group having 6 or more and 40 or less carbon atoms, 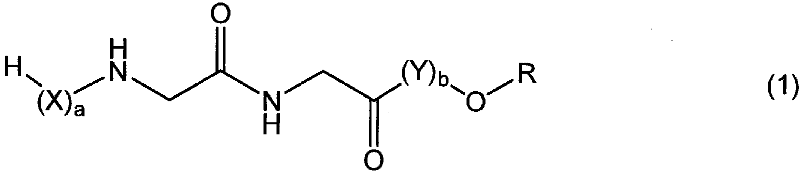
         wherein X represents a divalent hydrocarbon group having 1 to 4 carbon atoms and optionally substituted with hydroxyl group(s), or an amino acid residue, and
         Y represents an amino acid residue or a divalent group represented by formula (2): 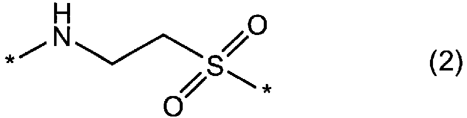
         wherein -* represents a bond to an adjacent carbonyl group or an oxygen atom,
         R represents a hydrogen atom or a monovalent hydrocarbon group having 1 to 4 carbon atoms and optionally substituted with hydroxyl group(s), and
         a and b represent 0 or 1, with the proviso that if a and b simultaneously represent 1, then X does not represent an amino acid residue;
      (B) a carboxylic acid having an inorganic value of 250 or more and 450 or less and an organic value of 50 or more and 250 or less, or a salt of the carboxylic acid, wherein the carboxylic acid is selected from the group consisting of a carboxylic acid represented by the following formula (B-1), malic acid, succinic acid, and lactic acid 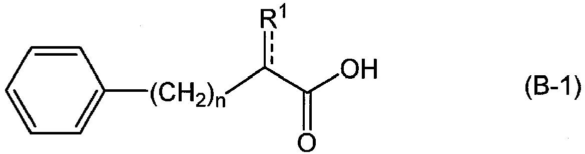 wherein
         R¹ represents a hydrogen atom, an oxygen atom, or a hydroxyl group;
         when R¹ is an oxygen atom, the dashed line indicates a double bond;
         n represents an integer from 0 - 3; and
         the phenyl group and methylene chain can be partly substituted with one or more hydroxyl groups;
   step (ii): a step of leaving the hair to stand at 15°C or more and 100°C or less for 15 seconds or more and 60 minutes or less, after step (i); and
   step (iii): a step of applying a hair treatment agent containing the following component (C) to the hair, after step (ii) :
      (C) an aromatic sulfonic acid having a molecular weight of 300 or less, or a salt thereof,
   wherein the method does not comprise applying a hair treatment agent containing a reducing agent.
<2> The hair treatment method according to <1>, wherein
   the hair treatment agent containing the components (A) and (B) is composed of a single composition containing the components (A) and (B) .
<3> The hair treatment method according to <2>, wherein the hair treatment agent containing the components (A) and (B) is a one-part hair treatment agent containing the components (A) and (B).
<4> The hair treatment method according to <1>, wherein the hair treatment agent containing the components (A) and (B) is composed of both a composition containing the component (A) and a composition containing the component (B) as constituents.
<5> The hair treatment method according to <4>, wherein the hair treatment agent containing the components (A) and (B) is preferably a multi-part hair treatment agent comprising a first part containing the component (A) and a second part containing the component (B) or a multi-part hair treatment agent comprising a first part containing the component (B) and a second part containing the component (A).
<6> The hair treatment method according to <5>, wherein step (i) is preferably carried out by applying the first part to hair, and thereafter applying the second part to the hair.
<7> The hair treatment method according to <6>, wherein preferably, step (i) further comprises step (i-cx) after the first part is applied to the hair and before the second part is applied to the hair:
   step (i-cx): a step of leaving the hair to stand at 15°C or more and 100°C or less for 15 seconds or more and 60 minutes or less.
<8> The hair treatment method according to any one of <1> to <7>, preferably, comprising a step of rinsing the hair after step (iii), and further comprising step (iv) between step (iii) and the step of rinsing the hair:
   step (iv): a step of leaving the hair, to which the hair treatment agent containing the component (C) is applied, at 15°C or more and 100°C or less for 1 minute or more and 60 minutes or less.
<9> The hair treatment method according to any one of <1> to <8>, wherein the glycylglycine derivative represented by formula (1) as component (A) is preferably a compound represented by any one of formulas (G1) to (G10), more preferably a compound represented by any one of formulas (G3) to (G10) and further preferably a compound represented by formula (G9) or (G10). 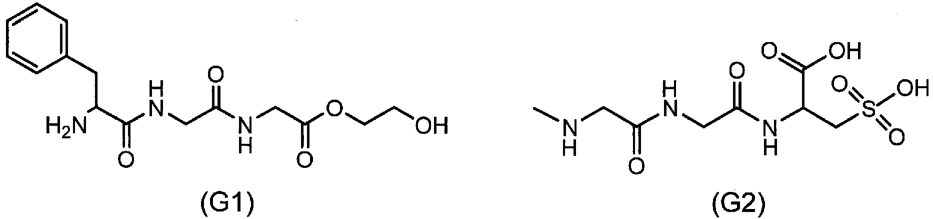 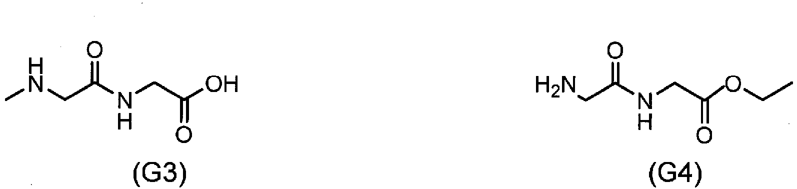 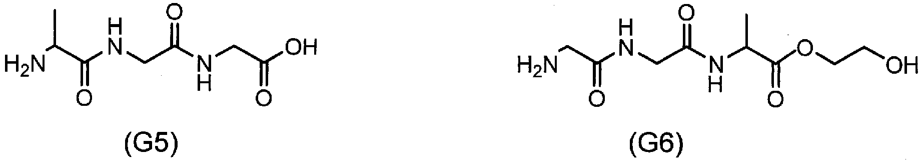 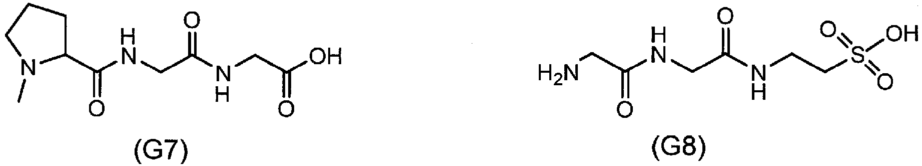 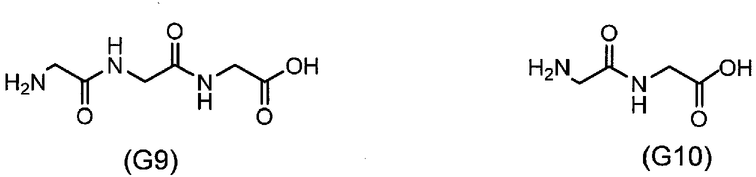
<10> The hair treatment method according to any one of <1> to <8>, wherein the component (A) is preferably a polyglycerin having an average degree of polymerization of 2 or more and 4 or less and more preferably an average degree of polymerization of 2 or more and 3 or less.
<11> The hair treatment method according to any one of <1> to <8>, wherein component (A) is an alkyl glyceryl ether having an alkyl group having preferably 8 or more and more preferably 14 or more, and preferably 20 or less and more preferably 18 or less carbon atoms.
<12> The hair treatment method according to any one of <2> to <11>, wherein the content of the component (A) in the composition containing the component (A) is preferably 0.5 mass% or more, more preferably 0.8 mass% or more, further preferably 1 mass% or more, further preferably 1.5 mass% or more, and further preferably 2.0 mass% or more; and preferably 10 mass% or less, more preferably 8 mass% or less, and further preferably 6 mass% or less.
<13> The hair treatment method according to any one of <1> to <12>, wherein the inorganic value of the component (B) as free acid is preferably 260 or more and more preferably 265 or more; and preferably 420 or less and more preferably 400 or less, and the organic value thereof is preferably 60 or more and more preferably 80 or more; and preferably 200 or less and more preferably 180 or less.
<14> The hair treatment method according to any one of <1> to <13>, wherein the component (B) is preferably at least one selected from the group consisting of mandelic acid, phenyllactic acid, malic acid, succinic acid, lactic acid, and salts of these, more preferably at least one selected from the group consisting of malic acid, mandelic acid, phenyllactic acid, and salts of these, further preferably at least one selected from the group consisting of malic acid, phenyllactic acid, and salts of these, further preferably malic acid or a salt thereof, and further preferably malic acid.
<15> The hair treatment method according to any one of <2> to <14>, wherein the content of component (B) contained in the composition containing the component (B) is preferably 1 mass% or more, more preferably 2.5 mass% or more, further preferably 5 mass% or more, further preferably 7.5 mass% or more, and further preferably 10 mass% or more; and preferably 25 mass% or less, more preferably 20 mass% or less, and further preferably 15 mass% or less.
<16> The hair treatment method according to any one of <2> to <15>, wherein the content of an organic solvent (F) represented by the following formula (F-1) in the composition containing the component (B), is preferably less than 5 mass%, more preferably less than 2 mass%, further preferably less than 1 mass%, further preferably less than 0.5 mass%, and further preferably 0 mass%,

   R²- (OCH₂CH₂)_{q}-R³ (F-1)

   wherein R² represents a hydrogen atom or an alkyl group having 1 or more and 5 or less carbon atoms; R³ represents a hydrogen atom or hydroxyl group(s); and q represents an integer of 0 or more and 5 or less, with the proviso that if q is 0, then R² and R³ are not hydrogen atoms.
<17> The hair treatment method according to any one of <2> to <16>, wherein the content of an aromatic alcohol (E) in the composition containing the component (B) is preferably less than 5 mass%, more preferably less than 2 mass%, further preferably less than 1 mass%, and further preferably 0 mass%.
<18> The hair treatment method according to any one of <2> to <17>, preferably, wherein the composition containing the component (A) and/or the component (B) further contains a thickener as component (D).
<19> The hair treatment method according to <18>, wherein the component (D) is preferably a natural or semi-synthetic polysaccharide, more preferably, at least one selected from the group consisting of xanthan gum, hydroxy xanthan gum, and hydroxyethyl cellulose, and further preferably at least one selected from the group consisting of xanthan gum and hydroxy xanthan gum.
<20> The hair treatment method according to <18> or <19>, wherein, the content of the component (D) in the composition containing the component (A) and/or the component (B) is preferably 0.1 mass% or more, more preferably 0.5 mass% or more, and further preferably 1 mass% or more; and preferably 5 mass% or less and more preferably 3 mass% or less.
<21> The hair treatment method according to any one of <2> to <20>, wherein pH of the composition containing the component (A) (excluding the composition containing the component (B)) at 25°C is preferably 7.5 or less, more preferably 7.0 or less, further preferably 6.5 or less, and further preferably 6.0 or less; and preferably 2 or more, more preferably 2.5 or more, and further preferably 2.8 or more.
<22> The hair treatment method according to any one of <2> to <21>, wherein pH of the composition containing the component (B) (excluding the composition containing the component (A)) at 25°C is preferably 2 or more, more preferably 3 or more, further preferably 3.5 or more, further preferably 4 or more, and further preferably 5 or more; and preferably 10 or less, more preferably 9 or less, and further preferably 8 or less.
<23> The hair treatment method according to <22>, wherein the pH of the composition containing the component (B) (excluding the composition containing the component (A)) at 25°C is further preferably beyond 5 and further preferably 6 or more.
<24> The hair treatment method according to <22>, wherein the pH of the composition containing the component (B) (excluding the composition containing component (A)) at 25°C is further preferably 7 or less and further preferably 5 or less; and further preferably 3 or more and further preferably 4 or more.
<25> The hair treatment method according to any one of <2> to <20>, wherein the pH of the composition containing the components (A) and (B) is preferably 2 or more, more preferably 2.5 or more, and further preferably 2.8 or more; and preferably 10 or less, more preferably 9 or less, and further preferably 8 or less.
<26> The hair treatment method according to any one of <1> to <25>, wherein the component (C) is preferably at least one selected from the group consisting of naphthalene sulfonic acid, azulene sulfonic acid, benzophenone sulfonic acid, and benzene sulfonic acid.
<27> The hair treatment method according to <26>, wherein the naphthalene sulfonic acid is preferably a compound represented by the following formula (3): 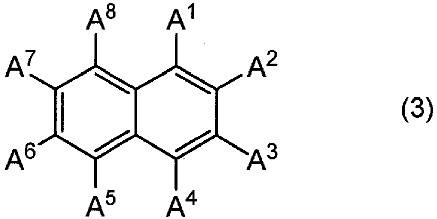 wherein one or more of A¹ to A⁸ represent a sulfo group or a salt thereof, the rest represent a hydrogen atom, a halogen atom, hydroxyl group(s), a nitro group, a carboxy group, a lower alkoxycarbonyl group, an alkyl group, an alkenyl group, a lower alkoxy group, a formyl group, an acyl group, a phenylazo group optionally having a substituent or -N(R')(R'') wherein R' and R'' represent a hydrogen atom, a lower alkyl group, a lower alkenyl group, a phenyl group, a benzyl group, or an acyl group.
<28> The hair treatment method according to <26>, wherein the benzophenone sulfonic acid is preferably a compound represented by the following formula (4): 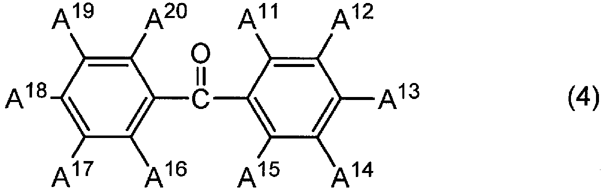 wherein one or more of A¹¹ to A²⁰ represent a sulfo group or a salt thereof, the rest represent a hydrogen atom, a halogen atom, hydroxyl group(s), a carboxy group, an amino group, a lower alkyl group, a lower alkenyl group, a lower alkoxy group, or an acyl group.
<29> The hair treatment method according to <26>, wherein the benzene sulfonic acid is preferably a compound represented by the following formula (5): 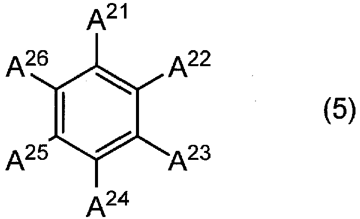 wherein one or more of A²¹ to A²⁶ represent a sulfo group or a salt thereof and the rest represent a hydrogen atom or a lower alkyl group.
<30> The hair treatment method according to <26>, wherein component (C) is preferably at least one selected from the group consisting of 2-naphthalene sulfonic acid, 1-naphthalene sulfonic acid, p-toluenesulfonic acid, xylene sulfonic acid, hydroxymethoxy benzophenone sulfonic acid, and salts of these and more preferably 1-or 2-naphthalene sulfonic acid or a salt thereof.
<31> The hair treatment method according to any one of <1> to <30>, wherein the content of the component (C) in the hair treatment agent to be used in step (iii) is preferably 1 mass% or more, more preferably 2.5 mass% or more, further preferably 4 mass% or more, further preferably 5 mass% or more, and further preferably 7 mass% or more; and preferably 25 mass% or less, more preferably 20 mass% or less, further preferably 15 mass% or less, and further preferably 10 mass% or less.
<32> The hair treatment method according to any one of <1> to <31>, wherein preferably, the hair treatment agent to be used in step (iii) further contains a thickener as the component (D).
<33> The hair treatment method according to <32>, wherein the component (D) is preferably a natural or semi-synthetic polysaccharide, more preferably at least one selected from the group consisting of xanthan gum, hydroxy xanthan gum, and hydroxyethyl cellulose, and further preferably at least one selected from the group consisting of xanthan gum and hydroxy xanthan gum.
<34> The hair treatment method according to <32> or <33>, wherein the content of the component (D) in the hair treatment agent to be used in step (iii) is preferably 0.1 mass% or more, more preferably 0.5 mass% or more, and further preferably 1 mass% or more; and preferably 5 mass% or less and more preferably 3 mass% or less.
<35> The hair treatment method according to any one of <1> to <34>, wherein preferably, the hair treatment agent to be used in step (iii) further contains an aromatic alcohol as component (E).
<36> The hair treatment method according to <35>, wherein the component (E) is preferably at least one selected from the group consisting of benzyl alcohol, cinnamyl alcohol, phenethyl alcohol, p-anisyl alcohol, p-methylbenzyl alcohol, phenoxyethanol, and 2-(benzyloxy)ethanol, more preferably at least one selected from the group consisting of benzyl alcohol and 2-(benzyloxy)ethanol, and further preferably benzyl alcohol.
<37> The hair treatment method according to <35> or <36>, wherein the content of the component (E) in the hair treatment agent to be used in step (iii) is preferably 1 mass% or more, more preferably 2 mass% or more, and further preferably 4 mass% or more; and preferably 30 mass% or less, more preferably 20 mass% or less, further preferably 15 mass% or less, and further preferably 10 mass% or less.
<38> The hair treatment method according to any one of <1> to <37>, wherein preferably, the hair treatment agent to be used in step (iii) further contains a polyhydric alcohol as component (G).
<39> The hair treatment method according to <38>, wherein the component (G) is preferably at least one selected from the group consisting of propylene glycol, dipropylene glycol, and polypropylene glycol.
<40> The hair treatment method according to <38> or <39>, wherein the content of the component (G) in the hair treatment agent to be used in step (iii) is preferably 5 mass% or more, more preferably 10 mass% or more, and further preferably 15 mass% or more; and preferably 30 mass% or less, more preferably 25 mass% or less, and further preferably 20 mass% or less.
<41> The hair treatment method according to any one of <1> to <40>, wherein pH of a 10-fold dilution solution of the hair treatment agent to be used in step (iii) with deionized water at 25°C is preferably 3 or more and more preferably 3.5 or more; and preferably 10 or less and more preferably 8 or less.
<42> The hair treatment method according to any one of <8> to <41>, wherein the temperature of leaving the hair to stand in step (iv) is 15°C or more; and preferably 60°C or less and more preferably 30°C or less.
<43> The hair treatment method according to <42>, wherein the temperature of leaving the hair to stand in step (iv) is preferably 15°C or more and less than 30°C.
<44> The hair treatment method according to any one of<8> to <41>, wherein the temperature of leaving the hair to stand in step (iv) is preferably 30°C or more and more preferably 40°C or more; and 100°C or less and more preferably 60°C or less, and the time of standing is 1 minute or more, preferably 3 minutes or more, more preferably 5 minutes or more; and preferably 30 minutes or less, more preferably 15 minutes or less, and further preferably 10 minutes or less.
<45> The hair treatment method according to any one of <1> to <44>, wherein preferably, the method does not comprise a step of applying either one of a hair treatment agent containing a reducing agent and a hair treatment agent of pH 12 to 14 to hair.
<46> A hair treatment method comprising the following steps (i-a) to (iv'):
   step (i-a): a step of applying a one-part hair treatment agent of pH 2 or more and 7.5 or less containing the following components (A), (B), and (D) and substantially not containing a hair reducing agent:
      (A) 1 mass% or more and 10 mass% or less of at least one compound selected from the group consisting of glycylglycine or a salt thereof, glycylglycylglycine or a salt thereof, a polyglycerin having an average degree of polymerization of 2 or more and 4 or less and an alkyl glyceryl ether having an alkyl group having 8 or more and 18 or less carbon atoms
      (B) 1 mass% or more and 15 mass% or less of a carboxylic acid having an inorganic value of 260 or more and 400 or less and an organic value of 60 or more and 180 or less, or a salt of the carboxylic acid, wherein the carboxylic acid is selected from the group consisting of a carboxylic acid represented by the following formula (B-1), malic acid, succinic acid, and lactic acid 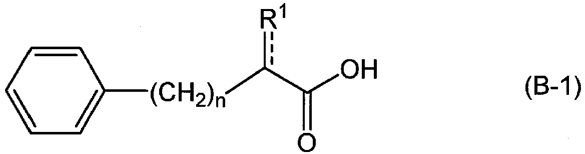 wherein
         R¹ represents a hydrogen atom, an oxygen atom, or a hydroxyl group;
         when R¹ is an oxygen atom, the dashed line indicates a double bond;
         n represents an integer from 0 - 3; and
         the phenyl group and methylene chain can be partly substituted with one or more hydroxyl groups;
      (D) 0.5 mass% or more and 3 mass% or less of a thickener
   step (ii): a step of leaving the hair to stand at 15°C or more and 60°C or less for 15 seconds or more and 45 minutes or less, after step (i-a);
   step (iii): a step of applying a hair treatment agent of pH 3 or more and 8 or less containing the following components (C), (D), and (E) and water and substantially not containing a hair reducing agent, to the hair, after step (ii):
      (C) 1 mass% or more and 15 mass% or less of at least one selected from the group consisting of 2-naphthalene sulfonic acid, 1-naphthalene sulfonic acid, p-toluenesulfonic acid, xylene sulfonic acid, hydroxymethoxy benzophenone sulfonic acid, and salts of these
      (D) 0.5 mass% or more and 3 mass% or less of a thickener
      (E) 4 mass% or more and 15 mass% or less of an aromatic alcohol;
   step (iv): a step of leaving the hair to stand at 15°C or more and 60°C or less for 3 minutes or more and 45 minutes or less, after step (iii);
   step (iv'): a step of rinsing the hair, after step (iv). [0209]
<47> A hair treatment method comprising the following steps (i-b) to (iv'):
   step (i-b): a step of mixing a first part and a second part of a multi-part hair treatment agent, which contains following component (A) as the first part and following component (B) as the second part or which contains the following component (A) as the second part and the following component (B) as the first part, which contains following component (D) in the first part and/or the second part, and which does not substantially contain a hair reducing agent, and applying the resultant mixture having pH 2 or more and 7.5 or less to hair:
      (A) 1 mass% or more and 10 mass% or less of at least one compound selected from the group consisting of glycylglycine or a salt thereof, glycylglycylglycine or a salt thereof, a polyglycerin having an average degree of polymerization of 2 or more and 4 or less, and an alkyl glyceryl ether having an alkyl group having 8 or more and 18 or less carbon atoms
      (B) 1 mass% or more and 15 mass% or less of a carboxylic acid having an inorganic value of 260 or more and 400 or less and an organic value of 60 or more and 180 or less, or a salt of the carboxylic acid, wherein the carboxylic acid is selected from the group consisting of a carboxylic acid represented by the following formula (B-1), malic acid, succinic acid, and lactic acid 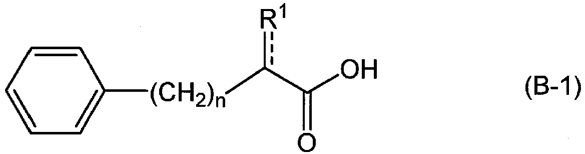 wherein
         R¹ represents a hydrogen atom, an oxygen atom, or a hydroxyl group;
         when R¹ is an oxygen atom, the dashed line indicates a double bond;
         n represents an integer from 0 - 3; and
         the phenyl group and methylene chain can be partly substituted with one or more hydroxyl groups
      (D) 0.5 mass% or more and 3 mass% or less of a thickener,
   step (ii): a step of leaving the hair to stand at 15°C or more and 60°C or less for 15 seconds or more and 45 minutes or less, after step (i-b);
   step (iii): a step of applying a hair treatment agent of pH 3 or more and 8 or less containing the following components (C), (D), (E), and water and substantially not containing a hair reducing agent to the hair, after step (ii):
      (C) 1 mass% or more and 15 mass% or less of at least one selected from the group consisting of 2-naphthalene sulfonic acid, 1-naphthalene sulfonic acid, p-toluenesulfonic acid, xylene sulfonic acid, hydroxymethoxy benzophenone sulfonic acid, and salts of these
      (D) 0.5 mass% or more and 3 mass% or less of a thickener;
      (E) 4 mass% or more and 15 mass% or less of an aromatic alcohol;
   step (iv): a step of leaving the hair to stand at 15°C or more and 60°C or less for 3 minutes or more and 45 minutes or less, after step (iii);
   step (iv'): a step of rinsing the hair after step (iv).
<48> A hair treatment method comprising the following steps (i-c-1) to (iv'):
   step (i-c-1): a step of applying a first part of a sequential-application type multi-part hair treatment agent of pH 2 or more and 7.5 or less containing the following component (A) and substantially not containing a hair reducing agent, to hair:
      (A) 1 mass% or more and 10 mass% or less of at least one compound selected from the group consisting of glycylglycine or a salt thereof, glycylglycylglycine or a salt thereof, a polyglycerin having an average degree of polymerization of 2 or more and 4 or less, and an alkyl glyceryl ether having an alkyl group having 8 or more and 18 or less carbon atoms;
   step (i-cx): a step of leaving the hair to stand at 15°C or more and 60°C or less for 15 seconds or more and 30 minutes or less, after step (i-c);
   step (i-c-2): a step of applying a second part of the sequential-application type multi-part hair treatment agent of pH 3.5 or more and 8 or less containing the following components (B) and (D) and substantially not containing a hair reducing agent, after step (i-cx):
      (B) 1 mass% or more and 15 mass% or less of a carboxylic acid having an inorganic value of 260 or more and 400 or less and an organic value of 60 or more and 180 or less, or a salt of the carboxylic acid, wherein the carboxylic acid is selected from the group consisting of a carboxylic acid represented by the following formula (B-1), malic acid, succinic acid, and lactic acid 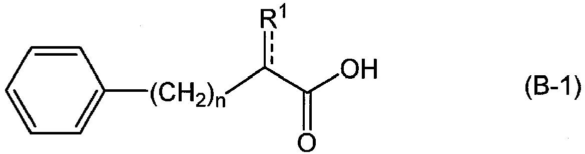 wherein
         R¹ represents a hydrogen atom, an oxygen atom, or a hydroxyl group;
         when R¹ is an oxygen atom, the dashed line indicates a double bond;
         n represents an integer from 0 - 3; and
         the phenyl group and methylene chain can be partly substituted with one or more hydroxyl groups
      (D) 0.5 mass% or more and 3 mass% or less of a thickener
   step (ii): a step of leaving the hair to stand at 15°C or more and 60°C or less for 15 seconds or more and 45 minutes or less, after step (i-c-2);
   step (iii): a step of applying a hair treatment agent of pH 3 or more and 8 or less containing the following components (C), (D), (E), and water and substantially not containing a hair reducing agent, after step (ii):
      (C) 1 mass% or more and 15 mass% or less of at least one selected from the group consisting of 2-naphthalene sulfonic acid, 1-naphthalene sulfonic acid, p-toluenesulfonic acid, xylene sulfonic acid, hydroxymethoxy benzophenone sulfonic acid, and salts of these
      (D) 0.5 mass% or more and 3 mass% or less of a thickener
      (E) 4 mass% or more and 15 mass% or less of an aromatic alcohol;
   step (iv): a step of leaving the hair to stand at 15°C or more and 60°C or less for 3 minutes or more and 45 minutes or less, after step (iii);
   step (iv'): a step of rinsing the hair, after step (iv).

### Examples

### [Tress for evaluation]

Using curly hair of Caucasian people having never been treated with a chemical agent, tresses having a length (at the time hair was pulled and linearly stretched) of 30 cm and a weight of 0.5 g were prepared. The tresses were washed with a model shampoo having the following composition, and thereafter, a model conditioner having the following composition was applied to the tresses. Then, the tresses were rinsed, wiped off with towel to remove extra water, and air-dried for 2 hours in laboratory conditions. The tresses thus prepared were used for evaluation.

### (Composition of model shampoo)

| Components | (mass%) |
|---|---|
| Sodium polyoxyethylene (2.5) lauryl ether sulfate | 15.5 |
| Lauric acid diethanolamide | 2.28 |
| Disodium edetate | 0.1 |
| Sodium benzoate | 0.5 |
| Oxybenzone | 0.03 |
| Phosphoric acid | 0.075 |
| Dibutylhydroxytoluene | 0.01 |
| Sodium chloride | 0.8 |
| Red No. 106 | 0.00012 |
| Fragrance | 0.26 |
| Purified water | balance |
| Total | 100 |

### (Composition of model conditioner)

| Component | (mass%) |
|---|---|
| Cetearyl alcohol | 2.0 |
| Steartrimonium chloride | 0.76 |
| Distearyldimonium chloride | 2.7 |
| Propylene glycol | 5.0 |
| Isopropanol | 0.6 |
| Ethyl paraben | 0.1 |
| Deionized water | balance |
| Total | 100 |

### [Examples 1 to 3]

### (One-part hair treatment agent containing components (A) and (B) + hair treatment agent containing component (C))

The one-part hair treatment agent for step (i) and a hair treatment agent for step (iii) shown in the following Table 1 were prepared. The tresses for evaluation were treated with these hair treatment agents in accordance with the following procedures and evaluated, in accordance with respective procedures for "wavy/curly hair relaxation effect", "durability of a wavy/curly hair relaxation effect against shampooing (once)", "durability of a wavy/curly hair relaxation effect against shampooing (30 times)", "smoothness of treated hair", "softness of the treated hair", and "less detangling of tress treated". The results are shown in Table 2.

Note that, pH of each hair treatment agent was measured at room temperature (25°C) by a pH meter (pH meter F-21 manufactured by HORIBA Ltd.). The one-part hair treatment agent for step (i) was directly measured; whereas the hair treatment agent for step (iii) was measured after it was diluted 10 times with deionized water. Note that, the "room temperature" described in Examples and Comparative Examples means 25°C.

**[Table 1]**

| | Component | | Mass% |
|---|---|---|---|
| One-part hair treatment agent for step (i) | (B) | Phenyllactic acid | 10 |
| | (D) | Xanthan gum | 1 |
| | (A) | Glycylglycine (*1) | 3 |
| | | Deionized water | Balance |
| | Total | | 100 |
| | pH | | 3 |
| Hair treatment agent for step (iii) | (C) | Sodium 2-naphthalene sulfonate | 5 |
| | (E) | Benzyl alcohol | 7.5 |
| | (G) | Dipropylene glycol | 20 |
| | (D) | Hydroxyethyl cellulose | 1 |
| | | Deionized water | Balance |
| | Total | | 100 |
| | pH | | 7 |

| | | | |
|---|---|---|---|
| ***1: compound represented by formula (G10)** | | | |

### (Treatment procedure)

### Example 1

To dry tresses for evaluation, 0.5 g of the one-part hair treatment agent for step (i) was applied and spread uniformly into the hair. The whole tresses were wrapped with a cover and sealed, and left to stand at room temperature for 15 minutes. Then, the cover was removed, further 0.5 g of the hair treatment agent for step (iii) was applied and spread into the hair without rinsing. The whole tresses were wrapped again with a cover and sealed, and left to stand at room temperature for 15 minutes.

Subsequently, the cover was removed, the tresses were rinsed with tap water of 40°C for 30 seconds. A model shampoo (1 g) was applied to the tresses and bubbled for 30 seconds. Thereafter, the tresses were rinsed for 30 seconds, and a model conditioner (1 g) was applied and spread into the hair for 30 seconds, rinsed out with tap water of 40°C for 30 seconds. Subsequently, the tresses dried with a towel and air-dried at room temperature for 2 hours.

### Example 2

To dry tresses for evaluation, 0.5 g of the one-part hair treatment agent for step (i) was applied and spread uniformly into the hair. The whole tresses were wrapped with a cover and sealed, and left to stand at room temperature for 15 minutes. Then, the cover was removed, further 0.5 g of the hair treatment agent for step (iii) was applied and spread into the tresses without rinsing. The whole tresses were wrapped again with a cover and sealed, and left to stand at room temperature for 15 minutes.

Subsequently, the cover was removed, the tresses were rinsed with tap water of 40°C for 30 seconds, dried with a towel and air-dried at room temperature for 2 hours.

### Example 3

To dry tresses for evaluation, 0.5 g of the one-part hair treatment agent for step (i) was applied and spread uniformly into the hair. The whole tresses were wrapped with a cover and sealed, and left to stand at room temperature for 15 minutes. Then, the cover was removed, further 0.5 g of the hair treatment agent for step (iii) was applied and spread into the hair without rinsing. The whole tresses were wrapped again with a cover and sealed, and left to stand at room temperature for 15 minutes.

Subsequently, the cover was removed, the hair treatment agent on the tresses was scraped off by a comb. The tresses were heated by a straight iron (model No. TITANIUM 450 manufactured by Taiff) heated to 200°C, and then, rinsed with tap water of 40°C for 30 seconds. A model shampoo (1 g) was applied and bubbled for 30 seconds, and then, rinsed out for 30 seconds. Thereafter, a model conditioner (1 g) was applied and spread into the hair for 30 seconds, rinsed out with tap water of 40°C for 30 seconds. Subsequently, the tresses were dried with a towel and airdried at room temperature for 2 hours.

### (Evaluation criteria)

### <Wavy/Curly hair relaxation effect>

Provided that the length of hair (at the time hair was pulled and linearly stretched) of a tress before subjected to the above hair treatment, was represented by L (30 cm), the length of the tress for evaluation after air-dried and before subjected to the treatment was represented by L₀, and the length of a tress for evaluation treated and air-dried was represented by L₁, the wavy/curly hair relaxation degree (%) was obtained in accordance with the following Expression 1. Note that, L₀ and L₁ are lengths measured while suspending tresses from above without stretching. The larger the numerical value is, more excellent the wavy/curly hair relaxation effect is. The results are shown in Table 2. Wavy/Curly hair relaxation degree = (L1 - L0) / (L - L0 × 100

### <Durability of wavy/curly hair relaxation effect against shampooing (once)>

The treated hair was made wet with tap water of 40°C for 30 seconds and the model shampoo (1 g) was applied to the hair, bubbled for 30 seconds, and rinsed out for 30 seconds. Then, the model conditioner (1 g) was applied, spread into the hair for 30 seconds, and rinsed out with tap water of 40°C for 30 seconds. Then the hair was dried with a towel, and then, with a hairdryer (EH646 manufactured by National) for 2 minutes. The treatment procedure is specified as a single hair-shampooing process.

The treated hair was soaked in water for 3 minutes, and then, dried with a towel and airdried at room temperature for 2 hours.

Provided that the length of tress shampooed and air-dried is represented by L₂, the sustained wavy/curly hair relaxation rate (%) was obtained in accordance with the following Expression 2. Note that, L₂ is a length measured while suspending tresses from above without stretching. The larger the numerical value is, the more excellent the sustained wavy/curly hair relaxation rate is. The results are shown in Table 2 Sustained wavy/curly hair relaxation rate = (L2 - L0) / (L1 - L0) × 100

### <Durability of wavy/curly hair relaxation effect against shampooing (30 times)>

The treatment procedure described in the section of "durability of a wavy/curly hair relaxation effect against shampooing (once)" was regarded as a single hair-shampooing process. The hair-shampooing process was repeated 30 times. The treated hair was soaked in water for 3 minutes, and then, dried with a towel and air-dried at room temperature for 2 hours.

The sustained wavy/curly hair relaxation rate (%) was obtained in accordance with Expression 2. The results are shown in Table 2.

### <Smoothness of the treated hair>

Smoothness of the treated hair was evaluated by 7 panels in comparison with reference hair treated with a model treatment process shown below. The 7 panels were required to select one of the evaluation criteria: "smoother than references hair", "equivalent", and "heavier than references hair". The number of panels who answered (selected) "smoother than references hair", "equivalent", and "heavier than references hair" are shown in this order.

A Reference tress treated with a model treatment was prepared by washing a tress for evaluation with the model shampoo, and thereafter, applying a model treatment (1 g) having the following composition, to the tress, leaving the tress to stand for 30 minutes, rinsing it with tap water of 40°C for 30 seconds, wiping off extra moisture with a towel, and air-drying it at room temperature for 2 hours, and then, subjected to evaluation.

### (Composition of model treatment)

| Component | (mass%) |
|---|---|
| Stearyl alcohol | 6.9 |
| Stearoxypropyldimethylamine (*1) | 2.0 |
| Sunflower seed oil | 1.5 |
| Hydrogenated castor oil hydroxystearate (*2) | 0.1 |
| Hexa (hydroxystearic acid/stearic acid/rosin acid) dipentaerythrityl (*3) | 0.4 |
| Lanolin fatty acid | 0.18 |
| Dimethicone | 3.2 |
| Amodimethicone | 0.3 |
| (Bisisobutyl PEG-15/amodimethicone) copolymer | 0.08 |
| Lactic acid | 1.3 |
| Dipropylene glycol | 3.5 |
| Benzyl alcohol | 0.4 |
| Fragrance | 0.45 |
| Deionized water | balance |
| Total | 100 |

| | |
|---|---|
| *1: Farmin DM E-80 (manufactured by Kao Corporation) *2: Technor MH (manufactured by Yokoseki Yushi Kogyo Co., Ltd. ) *3: Cosmol 168ARV (manufactured by Nisshin Oillio) | |

### <Softness of hair>

Softness of the treated hair was evaluated by 7 panels. The 7 panels were required to select one of the evaluation criteria: "softer than hair before treatment", "equivalent", and "harder than hair before treatment". The number of panels who answered (selected) "softer than hair before treatment", "equivalent", and "harder than hair before treatment" are shown in this order.

### <Less detangling of hair>

Less detangling of treated hair was evaluated by 7 panels. The 7 panels were required to select one of the evaluation criteria: "less detangled compared with hair before treatment", "equivalent", and "more detangled compared with hair before treatment". The number of panels who answered (selected) "less detangled compared with hair before treatment", "equivalent", and "more detangled compared with hair before treatment" are shown in this order.

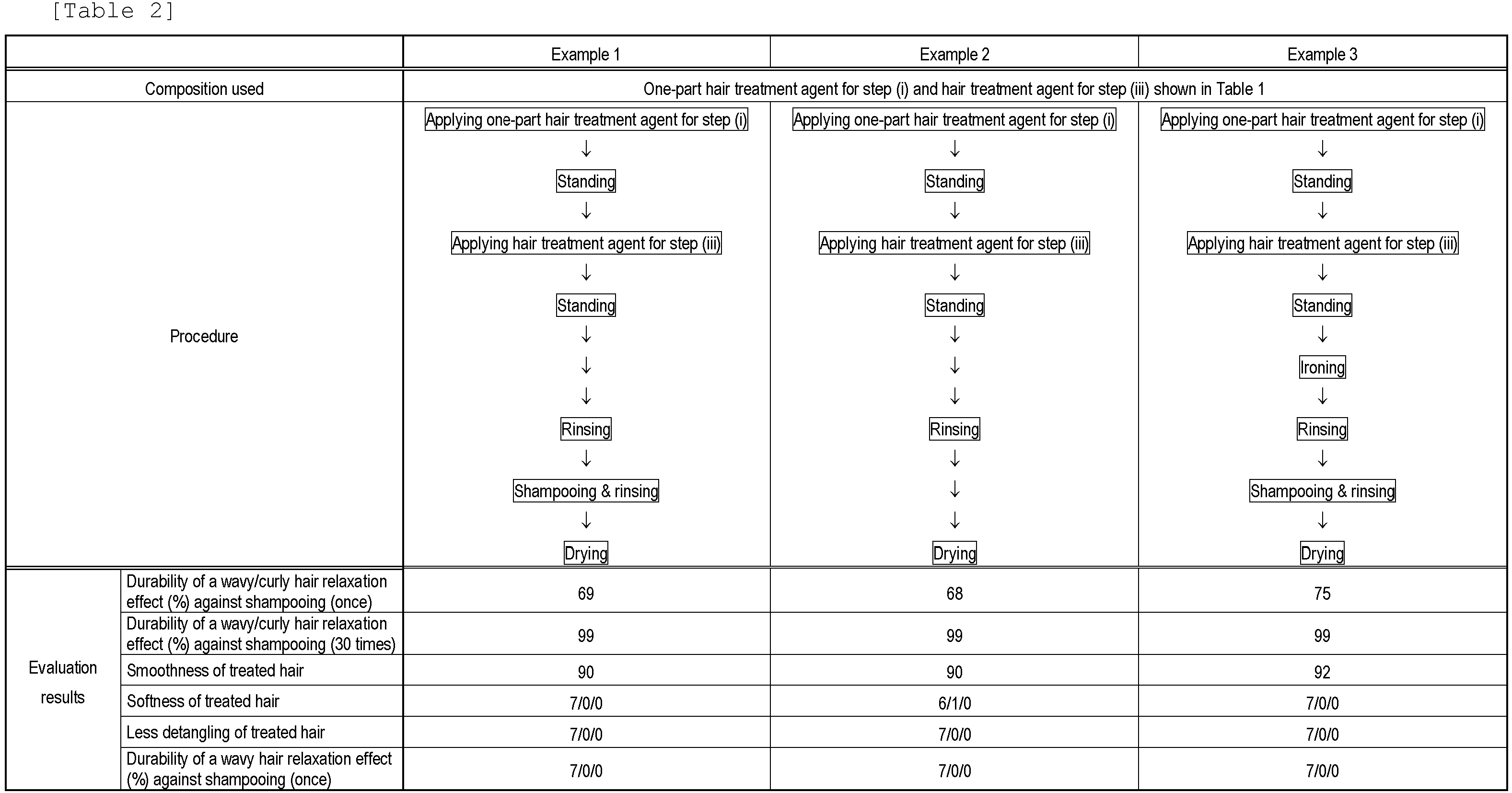

### [Examples 4 to 16]

### (One-part hair treatment agent containing components (A) and (B) + hair treatment agent containing component (C))

The one-part hair treatment agents for step (i) and the hair treatment agents for step (iii) of Examples 4 to 16 shown in Table 3 were prepared. Tresses for evaluation were treated in the same procedure as in Example 1 and subjected to evaluations: "wavy/curly hair relaxation effect", "durability of a wavy/curly hair relaxation effect against shampooing (once)", "durability of a wavy/curly hair relaxation effect against shampooing (30 times)", "smooth texture of treated hair", "softness of treated hair", and "less detangling of treated hair". The evaluation criteria were the same as shown above.

Note that, pH of each hair treatment agent was measured at room temperature (25°C) by a pH meter (pH meter F-21 manufactured by HORIBA Ltd). The one-part hair treatment agent for step (i) was directly measured; whereas the hair treatment agent for step (iii) was measured after it was diluted 10 times with deionized water.

**[Table 3]**

| (Unit of content is mass%) | | | Example | | | | | | | | | | | | |
|---|---|---|---|---|---|---|---|---|---|---|---|---|---|---|---|
| | | | 4 | 5 | 6 | 7 | 8 | 9 | 10 | 11 | 12 | 13 | 14 | 15 | 16 |
| One-part hair treatment agent for step (i) | (B) | Phenyllactic acid | - | - | - | - | - | - | - | - | - | - | - | - | 10 |
| | | Malic acid | 10 | 5 | 15 | 10 | 10 | 10 | 10 | 10 | 1.5 | - | 10 | 10 | - |
| | | Sodium malate | - | - | - | - | - | - | - | - | - | 10 | - | - | - |
| | (D) | Xanthan gum | 1 | 1 | 1 | 1 | 1 | 1 | 1 | 1 | 1 | 1 | 1 | 1 | 1 |
| | (A) | Glycylglycine (*1) | 3 | 3 | 3 | 1 | 5 | 3 | 3 | 3 | 3 | 3 | - | - | - |
| | | Polyglycerin-3 | - | - | - | - | - | - | - | - | - | - | 3 | - | - |
| | | Isostearyl glyceryl ether (*2) | - | - | - | - | - | - | - | - | - | - | - | 3 | 3 |
| | | Deionized water | Balance | Balance | Balance | Balance | Balance | Balance | Balance | Balance | Balance | Balance | Balance | Balance | Balance |
| | Total | | 100 | 100 | 100 | 100 | 100 | 100 | 100 | 100 | 100 | 100 | 100 | 100 | 100 |
| | pH | | 3 | 3 | 3 | 3 | 3 | 3 | 3 | 3 | 3 | 7 | 3 | 3 | 3 |
| Hair treatment agent for step (iii) | (C) | Sodium 2-naphthalene sulfonate | 5 | 5 | 5 | 5 | 5 | 3 | 7 | 5 | 5 | 5 | 5 | 5 | 5 |
| | (E) | Benzyl alcohol | 7.5 | 7.5 | 7.5 | 7.5 | 7.5 | 7.5 | 7.5 | 4 | 4 | 7.5 | 7.5 | 7.5 | 7.5 |
| | (G) | Dipropylene glycol | 20 | 20 | 20 | 20 | 20 | 20 | 20 | 20 | 20 | 20 | 20 | 20 | 20 |
| | (D) | Hydroxyethyl cellulose | 1 | 1 | 1 | 1 | 1 | 1 | 1 | 1 | 1 | 1 | 1 | 1 | 1 |
| | | Deionized water | Balance | Balance | Balance | Balance | Balance | Balance | Balance | Balance | Balance | Balance | Balance | Balance | Balance |
| | Total | | 100 | 100 | 100 | 100 | 100 | 100 | 100 | 100 | 100 | 100 | 100 | 100 | 100 |
| | pH | | 4 | 4 | 4 | 4 | 4 | 4 | 4 | 4 | 4 | 4 | 4 | 4 | 4 |
| Evaluation criteria | Wavy/curly hair relaxation effect | | 68 | 55 | 72 | 53 | 70 | 51 | 73 | 67 | 50 | 58 | 54 | 54 | 65 |
| | Durability of a wavy/curly hair relaxation effect against shampooing (once) | | 99 | 99 | 99 | 99 | 99 | 99 | 99 | 99 | 99 | 99 | 99 | 99 | 99 |
| | Durability of a wavy/curly hair relaxation effect against shampooing (30 times) | | 95 | 80 | 96 | 75 | 98 | 90 | 96 | 94 | 78 | 75 | 81 | 81 | 80 |
| | Smooth texture of treated hair | | 6/1/0 | 7/0/0 | 7/0/0 | 7/0/0 | 7/0/0 | 6/1/0 | 7/0/0 | 6/1/0 | 7/0/0 | 7/0/0 | 6/1/0 | 7/0/0 | 6/1/0 |
| | Soft texture of treated hair | | 7/0/0 | 6/1/0 | 7/0/0 | 6/1/0 | 6/1/0 | 6/1/0 | 6/1/0 | 7/0/0 | 6/1/0 | 7/0/0 | 7/0/0 | 7/0/0 | 6/1/0 |
| | Less detangling of treated hair | | 7/0/0 | 6/1/0 | 6/1/0 | 5/2/0 | 7/0/0 | 5/2/0 | 6/1/0 | 7/0/0 | 6/1/0 | 7/0/0 | 6/1/0 | 6/1/0 | 7/0/0 |

| | | | | | | | | | | | | | | | |
|---|---|---|---|---|---|---|---|---|---|---|---|---|---|---|---|
| *1: Compound represented by formula (G10) *2: PENETOL GE-IS (manufactured by KAO Corporation) | | | | | | | | | | | | | | | |

### [Example 17]

The first part and second part of the two-part hair treatment agent for step (i) and the hair treatment agent for step (iii) shown in the following Table 4 were prepared. Tresses for evaluation were treated with these hair treatment agents in accordance with the following procedure and evaluated in accordance with respective procedures for "wavy/curly hair relaxation effect", "durability of a wavy/curly hair relaxation effect against shampooing (once)", "durability of a wavy/curly hair relaxation effect against shampooing (30 times)", "smoothness of treated hair", "soft ness of treated hair", and "less detangling of the treated hair". The evaluation results are shown in Table 4.

Note that, pH of each hair treatment agent was evaluated at room temperature (25°C) by a pH meter (pH meter F-21 manufactured by HORIBA Ltd). The first part for step (i) and the second part for step (i) were directly measured; whereas the hair treatment agent for step (iii) was measured after it was diluted 10 times with deionized water.

### (Treatment procedure)

To dry tresses for evaluation, 0.5 g of the first part for step (i) was applied and spread uniformly into the hair. The whole tresses were wrapped with a cover and sealed, and left to stand at room temperature for 15 minutes. Then, the cover was removed, the tress was rinsed with tap water of 40°C for 10 seconds. Then, 0.5 g of the second part for step (i) was applied and spread into the hair. The whole tress was wrapped again with a cover and sealed, and left to stand at room temperature for 15 minutes. Then, further 0.5 g of the hair treatment agent for step (iii) was applied and spread into the hair without rinsing the tress. The whole tress was again wrapped with a cover and sealed, and left to stand at room temperature for 15 minutes.

The cover was removed, the tress was rinsed with tap water of 40°C for 30 seconds. A model shampoo (1 g) was applied to the tress and bubbled for 30 seconds. The tress was rinsed for 30 seconds and a model conditioner (1 g) was applied to the tress and spread into the hair for 30 seconds, rinsed out with tap water of 40°C for 30 seconds. Subsequently, the tress was dried with a towel and air-dried at room temperature for 2 hours.

**[Table 4]**

| (Unit of content is mass%) | | | Example |
|---|---|---|---|
| | | | 17 |
| First part for step (i) | (A) | Glycylglycine (*1) | 3 |
| | (E) | Benzyl alcohol | 4 |
| | | Hydrochloric acid | Amount to provide pH 3 |
| | | Deionized water | Balance |
| | Total | | 100 |
| | pH | | 3 |
| Second part for step (i) | (B) | Sodium malate | 10 |
| | (D) | Xanthan gum | 1 |
| | | Deionized water | Balance |
| | Total | | 100 |
| | pH | | 7 |
| Hair treatment agent for step (iii) | (C) | Sodium 2-naphthalene sulfonate | 5 |
| | (E) | Benzyl alcohol | 7.5 |
| | (G) | Dipropylene glycol | 20 |
| | (D) | Hydroxyethyl cellulose | 1 |
| | | Deionized water | Balance |
| | Total | | 100 |
| | pH | | 7 |
| Evaluation criteria | Wavy/curly hair relaxation effect | | 66 |
| | Durability of a wavy/curly hair relaxation effect against shampooing (once) | | 99 |
| | Durability of a wavy/curly hair relaxation effect against shampooing (30 times) | | 86 |
| | Smooth texture of treated hair | | 6/1/0 |
| | Softness of treated hair | | 6/1/0 |
| | Less detangling of treated hair | | 7/0/0 |

| | | | |
|---|---|---|---|
| ***1: Compound represented by formula (G10)** | | | |

### [Comparative Examples 1 and 2]

Hair treatment agents shown in the following Table 5 were prepared. Tresses for evaluation were treated with these hair treatment agents in accordance with the following procedure and evaluated in accordance with respective procedures for "wavy/curly hair relaxation effect", "durability of a wavy/curly hair relaxation effect against shampooing (once)", "durability of a wavy/curly hair relaxation effect against shampooing (30 times)", "smoothness of treated hair", "softness of treated hair", and "less detangling of treated hair". The evaluation results are shown in Table 5.

Note that, pH of each hair treatment agent was evaluated at room temperature (25°C) by a pH meter (pH meter F-21 manufactured by HORIBA Ltd). The agent of Comparative Example 1 was directly measured; whereas the agent of Comparative Example 2 was measured after it was diluted 10 times with deionized water.

### (Treatment procedure)

To dry tresses for evaluation, 0.5 g of the hair treatment agent was applied and spread uniformly into the hair. The whole tresses were wrapped with a cover and sealed, and left to stand at room temperature for 15 minutes.

Then, the cover was removed, the tress was rinsed with tap water of 40°C for 30 seconds. A model shampoo (1 g) was applied to the tress and bubbled for 30 seconds. The tress was rinsed for 30 seconds and a model conditioner (1 g) was applied to the tress and spread into the hair for 30 seconds, rinsed out with tap water of 40°C for 30 seconds. Subsequently, the tress was dried with a towel and air-dried at room temperature for 2 hours.

**[Table 5]**

| (Unit of content is mass%) | | | Comparative Example | |
|---|---|---|---|---|
| | | | 1 | 2 |
| Hair treatment agent | (A) | Glycylglycine (*1) | 3 | 3 |
| | (C) | Sodium 2-naphthalene sulfonate | - | 5 |
| | (E) | Benzyl alcohol | - | 7.5 |
| | (G) | Dipropylene glycol | - | 20 |
| | (D) | Hydroxyethyl cellulose | - | 1 |
| | | Xanthan gum | 1 | - |
| | | Deionized water | Balance | Balance |
| | Total | | 100 | 100 |
| | pH | | 3 | 3 |
| Evaluation criteria | Wavy/curly hair relaxation effect | | 0 | 4 |
| | Durability of a wavy/curly hair relaxation effect against shampooing (once) | | * | 0 |
| | Durability of a wavy/curly hair relaxation effect against shampooing (30 times) | | * | 0 |
| | Smoothness of treated hair | | 0/2/5 | 2/4/1 |
| | Softness of treated hair | | 6/1/0 | 6/1/0 |
| | Less detangling of treated hair | | 1/6/0 | 1/6/0 |

| | | | | |
|---|---|---|---|---|
| ***1: Compound represented by formula (G10)** *** Not evaluated since no wavy/curly hair relaxation effect was obtained** | | | | |

## Claims

1. A hair treatment method comprising the following steps (i) to (iii):
step (i): a step of applying a hair treatment agent containing the following components (A) and (B) to hair:
(A) at least one compound selected from the group consisting of a glycylglycine derivative represented by formula (1) or a salt thereof, a polyglycerin having an average degree of polymerization of 2 or more and 6 or less, and an alkyl glyceryl ether having an alkyl group having 6 or more and 40 or less carbon atoms, 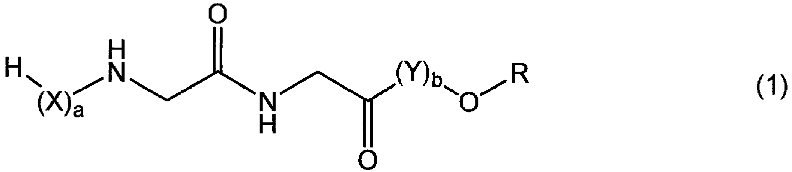 wherein X represents a divalent hydrocarbon group having 1 to 4 carbon atoms and optionally substituted with hydroxyl group(s), or an amino acid residue, and
Y represents an amino acid residue or a divalent group represented by formula (2): 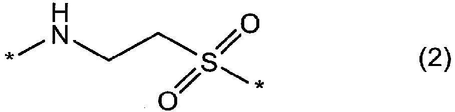 wherein -* represents a bond to an adjacent carbonyl group or an oxygen atom,
R represents a hydrogen atom or a monovalent hydrocarbon group having 1 to 4 carbon atoms and optionally substituted with hydroxyl group(s), and
a and b represent 0 or 1, with the proviso that if a and b simultaneously represent 1, then X does not represent an amino acid residue;
(B) a carboxylic acid having an inorganic value of 250 or more and 450 or less and an organic value of 50 or more and 250 or less, or a salt of the carboxylic acid, wherein the carboxylic acid is selected from the group consisting of a carboxylic acid represented by the following formula (B-1), malic acid, succinic acid, and lactic acid 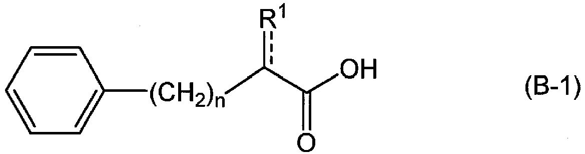 wherein
R¹ represents a hydrogen atom, an oxygen atom, or a hydroxyl group;
when R¹ is an oxygen atom, the dashed line indicates a double bond;
n represents an integer from 0 - 3; and
the phenyl group and methylene chain can be partly substituted with one or more hydroxyl groups;
step (ii): a step of leaving the hair to stand at 15°C or more and 100°C or less for 15 seconds or more and 60 minutes or less, after step (i); and
step (iii): a step of applying a hair treatment agent containing the following component (C) to the hair, after step (ii) :
(C) an aromatic sulfonic acid having a molecular weight of 300 or less, or a salt thereof,
wherein the method does not comprise applying a hair treatment agent containing a reducing agent.

2. The hair treatment method according to claim 1, wherein the hair treatment agent containing the components (A) and (B) is composed of a single composition containing the components (A) and (B), or is composed of both a composition containing the component (A) and a composition containing the component (B) as constituents.

3. The hair treatment method according to claim 1 or 2, wherein the hair treatment agent containing the components (A) and (B) is a one-part hair treatment agent containing the components (A) and (B).

4. The hair treatment method according to claim 1 or 2, wherein the hair treatment agent containing the components (A) and (B) is a multi-part hair treatment agent comprising a first part containing the component (A) and a second part containing the component (B) or a multi-part hair treatment agent comprising a first part containing the component (B) and a second part containing the component (A).

5. The hair treatment method according to claim 4, wherein step (i) is carried out by applying the first part to hair, and thereafter applying the second part to the hair.

6. The hair treatment method according to claim 5, wherein step (i) further comprises step (i-cx) after the first part is applied to the hair and before the second part is applied to the hair:
step (i-cx): a step of leaving the hair to stand at 15°C or more and 100°C or less for 15 seconds or more and 60 minutes or less.

7. The hair treatment method according to any one of claims 1 to 6, comprising a step of rinsing the hair after step (iii), and further comprising step (iv) between step (iii) and the step of rinsing the hair:
step (iv): a step of leaving the hair, to which the hair treatment agent containing the component (C) is applied, at 15°C or more and 100°C or less for 1 minute or more and 60 minutes or less.

8. The hair treatment method according to any one of claims 2 to 7, wherein the content of an organic solvent (F) represented by the following formula (F-1) in the composition containing the component (B) is less than 5 mass%,
R²-(OCH₂CH₂)_{q}-R³ (F-1)
wherein R² represents a hydrogen atom or an alkyl group having 1 or more and 5 or less carbon atoms; R³ represents a hydrogen atom or hydroxyl group(s); and q represents an integer of 0 or more and 5 or less, with the proviso that if q is 0, then R² and R³ are not hydrogen atoms.

9. The hair treatment method according to any one of claims 2 to 8, wherein the content of an aromatic alcohol (E) in the composition containing the component (B) is less than 5 mass%.

10. The hair treatment method according to any one of claims 2 to 9, wherein the composition containing the component (A) and/or the component (B) further contains a thickener as component (D).

11. The hair treatment method according to any one of claims 2 to 10, wherein the pH of the composition containing the component (B) at 25°C is 2 or more and 10 or less, provided that the composition does not contain the component (A).

12. The hair treatment method according to claim 11, wherein the pH of the composition containing the component (B) at 25°C is 5 or more and 8 or less, provided that the composition does not contain the component (A).

13. The hair treatment method according to any one of claims 2 to 10, wherein the pH of the composition containing the components (A) and (B) at 25°C is 2 or more and 10 or less.

14. The hair treatment method according to any one of claims 1 to 13, wherein the hair treatment agent containing the component (C) further contains a thickener as the component (D) and an aromatic alcohol as the component (E), and the content of the component (D) is 0.1 mass% to 0.5 mass%.

15. The hair treatment method according to any one of claims 7 to 14, wherein the temperature in step (iv) of leaving the hair to stand is 15°C or more and less than 30°C.

16. The hair treatment method according to any one of claims 7 to 14, wherein the temperature in step (iv) of leaving the hair to stand is 30°C or more and 100°C or less, and the time of leaving the hair to stand is 1 minute or more and 30 minutes or less.

17. The hair treatment method according to any one of claims 1 to 16, wherein the method does not comprise a step of applying a hair treatment agent of pH 12 to 14 to hair.

18. A hair treatment method comprising the following steps (i-a) to (iv'):
step (i-a): a step of applying a one-part hair treatment agent of pH 2 or more and 7.5 or less containing the following components (A), (B), and (D) and substantially not containing a hair reducing agent:
(A) 1 mass% or more and 10 mass% or less of at least one compound selected from the group consisting of glycylglycine or a salt thereof, glycylglycylglycine or a salt thereof, a polyglycerin having an average degree of polymerization of 2 or more and 4 or less and an alkyl glyceryl ether having an alkyl group having 8 or more and 18 or less carbon atoms
(B) 1 mass% or more and 15 mass% or less of a carboxylic acid having an inorganic value of 260 or more and 400 or less and an organic value of 60 or more and 180 or less, or a salt of the carboxylic acid, wherein the carboxylic acid is selected from the group consisting of a carboxylic acid represented by the following formula (B-1), malic acid, succinic acid, and lactic acid 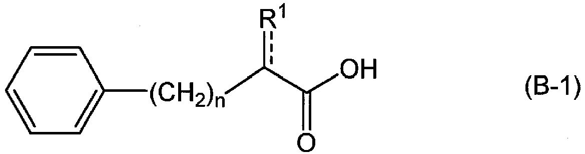 wherein
R¹ represents a hydrogen atom, an oxygen atom, or a hydroxyl group;
when R¹ is an oxygen atom, the dashed line indicates a double bond;
n represents an integer from 0 - 3; and
the phenyl group and methylene chain can be partly substituted with one or more hydroxyl groups;
(D) 0.5 mass% or more and 3 mass% or less of a thickener
step (ii): a step of leaving the hair to stand at 15°C or more and 60°C or less for 15 seconds or more and 45 minutes or less, after step (i-a);
step (iii): a step of applying a hair treatment agent of pH 3 or more and 8 or less containing the following components (C), (D), and (E) and water and substantially not containing a hair reducing agent, to the hair, after step (ii):
(C) 1 mass% or more and 15 mass% or less of at least one selected from the group consisting of 2-naphthalene sulfonic acid, 1-naphthalene sulfonic acid, p-toluenesulfonic acid, xylene sulfonic acid, hydroxymethoxy benzophenone sulfonic acid, and salts of these
(D) 0.5 mass% or more and 3 mass% or less of a thickener
(E) 4 mass% or more and 15 mass% or less of an aromatic alcohol;
step (iv): a step of leaving the hair to stand at 15°C or more and 60°C or less for 3 minutes or more and 45 minutes or less, after step (iii);
step (iv'): a step of rinsing the hair, after step (iv).

19. A hair treatment method comprising the following steps (i-b) to (iv'):
step (i-b): a step of mixing a first part and a second part of a multi-part hair treatment agent, which contains following component (A) as the first part and following component (B) as the second part or which contains the following component (A) as the second part and the following component (B) as the first part, which contains following component (D) in the first part and/or the second part, and which does not substantially contain a hair reducing agent, and applying the resultant mixture having pH 2 or more and 7.5 or less to hair:
(A) 1 mass% or more and 10 mass% or less of at least one compound selected from the group consisting of glycylglycine or a salt thereof, glycylglycylglycine or a salt thereof, a polyglycerin having an average degree of polymerization of 2 or more and 4 or less, and an alkyl glyceryl ether having an alkyl group having 8 or more and 18 or less carbon atoms
(B) 1 mass% or more and 15 mass% or less of a carboxylic acid having an inorganic value of 260 or more and 400 or less and an organic value of 60 or more and 180 or less, or a salt of the carboxylic acid, wherein the carboxylic acid is selected from the group consisting of a carboxylic acid represented by the following formula (B-1), malic acid, succinic acid, and lactic acid 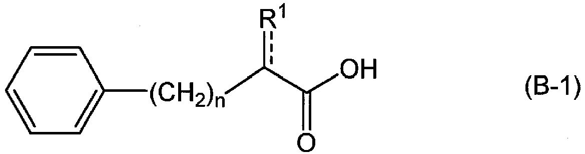 wherein
R¹ represents a hydrogen atom, an oxygen atom, or a hydroxyl group;
when R¹ is an oxygen atom, the dashed line indicates a double bond;
n represents an integer from 0 - 3; and
the phenyl group and methylene chain can be partly substituted with one or more hydroxyl groups
(D) 0.5 mass% or more and 3 mass% or less of a thickener,
step (ii): a step of leaving the hair to stand at 15°C or more and 60°C or less for 15 seconds or more and 45 minutes or less, after step (i-b);
step (iii): a step of applying a hair treatment agent of pH 3 or more and 8 or less containing the following components (C), (D), (E), and water and substantially not containing a hair reducing agent to the hair, after step (ii):
(C) 1 mass% or more and 15 mass% or less of at least one selected from the group consisting of 2-naphthalene sulfonic acid, 1-naphthalene sulfonic acid, p-toluenesulfonic acid, xylene sulfonic acid, hydroxymethoxy benzophenone sulfonic acid, and salts of these
(D) 0.5 mass% or more and 3 mass% or less of a thickener;
(E) 4 mass% or more and 15 mass% or less of an aromatic alcohol;
step (iv): a step of leaving the hair to stand at 15°C or more and 60°C or less for 3 minutes or more and 45 minutes or less, after step (iii);
step (iv'): a step of rinsing the hair after step (iv).

20. A hair treatment method comprising the following steps (i-c-1) to (iv'):
step (i-c-1): a step of applying a first part of a sequential-application type multi-part hair treatment agent of pH 2 or more and 7.5 or less containing the following component (A) and substantially not containing a hair reducing agent, to hair:
(A) 1 mass% or more and 10 mass% or less of at least one compound selected from the group consisting of glycylglycine or a salt thereof, glycylglycylglycine or a salt thereof, a polyglycerin having an average degree of polymerization of 2 or more and 4 or less, and an alkyl glyceryl ether having an alkyl group having 8 or more and 18 or less carbon atoms;
step (i-cx): a step of leaving the hair to stand at 15°C or more and 60°C or less for 15 seconds or more and 30 minutes or less, after step (i-c);
step (i-c-2): a step of applying a second part of the sequential-application type multi-part hair treatment agent of pH 3.5 or more and 8 or less containing the following components (B) and (D) and substantially not containing a hair reducing agent, after step (i-cx):
(B) 1 mass% or more and 15 mass% or less of a carboxylic acid having an inorganic value of 260 or more and 400 or less and an organic value of 60 or more and 180 or less, or a salt of the carboxylic acid, wherein the carboxylic acid is selected from the group consisting of a carboxylic acid represented by the following formula (B-1), malic acid, succinic acid, and lactic acid 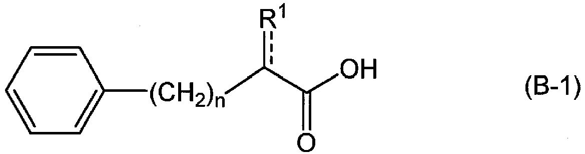 wherein
R¹ represents a hydrogen atom, an oxygen atom, or a hydroxyl group;
when R¹ is an oxygen atom, the dashed line indicates a double bond;
n represents an integer from 0 - 3; and
the phenyl group and methylene chain can be partly substituted with one or more hydroxyl groups
(D) 0.5 mass% or more and 3 mass% or less of a thickener
step (ii): a step of leaving the hair to stand at 15°C or more and 60°C or less for 15 seconds or more and 45 minutes or less, after step (i-c-2);
step (iii): a step of applying a hair treatment agent of pH 3 or more and 8 or less containing the following components (C), (D), (E), and water and substantially not containing a hair reducing agent, after step (ii):
(C) 1 mass% or more and 15 mass% or less of at least one selected from the group consisting of 2-naphthalene sulfonic acid, 1-naphthalene sulfonic acid, p-toluenesulfonic acid, xylene sulfonic acid, hydroxymethoxy benzophenone sulfonic acid, and salts of these
(D) 0.5 mass% or more and 3 mass% or less of a thickener
(E) 4 mass% or more and 15 mass% or less of an aromatic alcohol;
step (iv): a step of leaving the hair to stand at 15°C or more and 60°C or less for 3 minutes or more and 45 minutes or less, after step (iii);
step (iv'): a step of rinsing the hair, after step (iv).

## Patentansprüche

1. Haarbehandlungsverfahren, umfassend die folgenden Schritte (i) bis (iii):
Schritt (i): ein Schritt des Auftragens eines Haarbehandlungsmittels, enthaltend die folgenden Komponenten (A) und (B), auf das Haar:
(A) mindestens eine Verbindung, ausgewählt aus der Gruppe, bestehend aus einem Glycylglycinderivat, dargestellt durch Formel (1), oder einem Salz davon, einem Polyglycerin mit einem durchschnittlichen Polymerisationsgrad von 2 oder mehr und 6 oder weniger und einem Alkylglycerylether mit einer Alkylgruppe mit 6 oder mehr und 40 oder weniger Kohlenstoffatomen, 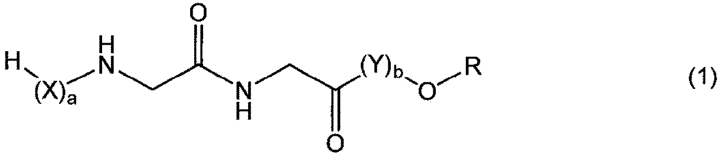
worin X für eine divalente Kohlenwasserstoffgruppe, die 1 bis 4 Kohlenstoffatome aufweist und optional mit Hydroxylgruppe(n) substituiert ist, oder einen Aminosäurerest steht, und
Y für einen Aminosäurerest oder eine divalente Gruppe, dargestellt durch Formel (2), steht: 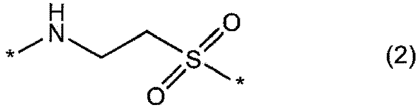
worin -* für eine Bindung zu einer benachbarten Carbonylgruppe oder einem Sauerstoffatom steht,
R für ein Wasserstoffatom oder eine monovalente Kohlenwasserstoffgruppe, die 1 bis 4 Kohlenstoffatome aufweist und optional mit Hydroxylgruppe(n) substituiert ist, steht, und
a und b für 0 oder 1 stehen, mit der Maßgabe, dass, wenn a und b gleichzeitig 1 bedeuten, X nicht für einen Aminosäurerest steht;
(B) eine Carbonsäure mit einem anorganischen Wert von 250 oder mehr und 450 oder weniger und einem organischen Wert von 50 oder mehr und 250 oder weniger oder ein Salz der Carbonsäure, wobei die Carbonsäure aus der Gruppe, bestehend aus einer Carbonsäure, dargestellt durch die folgende Formel (B-1), Äpfelsäure, Bernsteinsäure und Milchsäure, ausgewählt ist 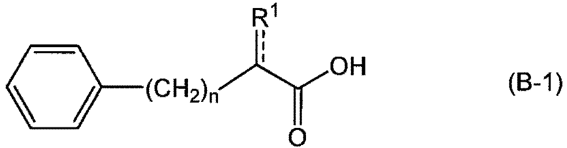 worin
R¹ für ein Wasserstoffatom, ein Sauerstoffatom oder eine Hydroxylgruppe steht;
wenn R¹ ein Sauerstoffatom ist, die gestrichelte Linie eine Doppelbindung anzeigt;
n für eine ganze Zahl von 0 bis 3 steht; und
die Phenylgruppe und die Methylenkette teilweise mit einer oder mehreren Hydroxylgruppen substituiert sein können;
Schritt (ii): ein Schritt des Belassens des Haars bei 15°C oder mehr und 100°C oder weniger für 15 Sekunden oder mehr und 60 Minuten oder weniger nach Schritt (i); und
Schritt (iii): ein Schritt des Auftragens eines Haarbehandlungsmittels, enthaltend die folgende Komponente (C), auf das Haar nach Schritt (ii):
(C) eine aromatische Sulfonsäure mit einem Molekulargewicht von 300 oder weniger oder ein Salz davon,
wobei das Verfahren nicht das Auftragen eines Haarbehandlungsmittels, das ein Reduktionsmittel enthält, umfasst.

2. Haarbehandlungsverfahren gemäß Anspruch 1, wobei das Haarbehandlungsmittel, das die Komponenten (A) und (B) enthält, aus einer einzigen Zusammensetzung besteht, die die Komponenten (A) und (B) enthält, oder aus einer Zusammensetzung, enthaltend die Komponente (A), und einer Zusammensetzung, enthaltend die Komponente (B), als Bestandteile besteht.

3. Haarbehandlungsverfahren gemäß Anspruch 1 oder 2, wobei das Haarbehandlungsmittel, das die Komponenten (A) und (B) enthält, ein einteiliges Haarbehandlungsmittel ist, das die Komponenten (A) und (B) enthält.

4. Haarbehandlungsverfahren gemäß Anspruch 1 oder 2, wobei das Haarbehandlungsmittel, das die Komponenten (A) und (B) enthält, ein mehrteiliges Haarbehandlungsmittel ist, das einen ersten Teil, der die Komponente (A) enthält, und einen zweiten Teil, der die Komponente (B) enthält, umfasst oder ein mehrteiliges Haarbehandlungsmittel ist, das einen ersten Teil, der die Komponente (B) enthält, und einen zweiten Teil, der die Komponente (A) enthält, umfasst.

5. Haarbehandlungsverfahren gemäß Anspruch 4, wobei Schritt (i) durch Auftragen des ersten Teils auf das Haar und anschließendes Auftragen des zweiten Teils auf das Haar durchgeführt wird.

6. Haarbehandlungsverfahren gemäß Anspruch 5, wobei Schritt (i) ferner Schritt (i-cx) umfasst, nachdem der erste Teil auf das Haar aufgetragen wurde und bevor der zweite Teil auf das Haar aufgetragen wird:
Schritt (i-cx): ein Schritt des Belassens des Haars bei 15°C oder mehr und 100°C oder weniger für 15 Sekunden oder mehr und 60 Minuten oder weniger.

7. Haarbehandlungsverfahren gemäß einem der Ansprüche 1 bis 6, umfassend einen Schritt des Ausspülens des Haars nach Schritt (iii) und ferner umfassend Schritt (iv) zwischen Schritt (iii) und dem Schritt des Ausspülens des Haars:
Schritt (iv): ein Schritt des Belassens des Haars, auf das das Haarbehandlungsmittel, das die Komponente (C) enthält, aufgetragen wurde, bei 15°C oder mehr und 100°C oder weniger für 1 Minute oder mehr und 60 Minuten oder weniger.

8. Haarbehandlungsverfahren gemäß einem der Ansprüche 2 bis 7, wobei der Gehalt an einem organischen Lösungsmittel (F), dargestellt durch die folgende Formel (F-1), in der Zusammensetzung, die die Komponente (B) enthält, weniger als 5 Massen-% beträgt,
R²-(OCH₂CH₂)_{q}-R³ (F-1)
worin R² für ein Wasserstoffatom oder eine Alkylgruppe mit 1 oder mehr und 5 oder weniger Kohlenstoffatomen steht; R³ für ein Wasserstoffatom oder Hydroxylgruppe(n) steht; und q für eine ganze Zahl von 0 oder mehr und 5 oder weniger steht, mit der Maßgabe, dass, wenn q 0 ist, R² und R³ keine Wasserstoffatome sind.

9. Haarbehandlungsverfahren gemäß einem der Ansprüche 2 bis 8, wobei der Gehalt eines aromatischen Alkohols (E) in der Zusammensetzung, die die Komponente (B) enthält, weniger als 5 Massen-% beträgt.

10. Haarbehandlungsverfahren gemäß einem der Ansprüche 2 bis 9, wobei die Zusammensetzung, die die Komponente (A) und/oder die Komponente (B) enthält, weiterhin ein Verdickungsmittel als Komponente (D) enthält.

11. Haarbehandlungsverfahren gemäß einem der Ansprüche 2 bis 10, wobei der pH-Wert der Zusammensetzung, die Komponente (B) enthält, bei 25°C 2 oder mehr und 10 oder weniger beträgt, mit der Maßgabe, dass die Zusammensetzung nicht die Komponente (A) enthält.

12. Haarbehandlungsverfahren gemäß Anspruch 11, wobei der pH-Wert der Zusammensetzung, die die Komponente (B) enthält, bei 25°C 5 oder mehr und 8 oder weniger beträgt, mit der Maßgabe, dass die Zusammensetzung nicht die Komponente (A) enthält.

13. Haarbehandlungsverfahren gemäß einem der Ansprüche 2 bis 10, wobei der pH-Wert der Zusammensetzung, die die Komponenten (A) und (B) enthält, bei 25°C 2 oder mehr und 10 oder weniger beträgt.

14. Haarbehandlungsverfahren gemäß einem der Ansprüche 1 bis 13, wobei das Haarbehandlungsmittel, das die Komponente (C) enthält, ferner ein Verdickungsmittel als Komponente (D) und einen aromatischen Alkohol als Komponente (E) enthält und der Gehalt an der Komponente (D) 0,1 Massen-% bis 0,5 Massen-% beträgt.

15. Haarbehandlungsverfahren gemäß einem der Ansprüche 7 bis 14, wobei die Temperatur in Schritt (iv) des Belassens des Haars 15°C oder mehr und weniger als 30°C beträgt.

16. Haarbehandlungsverfahren gemäß einem der Ansprüche 7 bis 14, wobei die Temperatur in Schritt (iv) des Belassens des Haars 30°C oder mehr und 100°C oder weniger beträgt, und die Zeit des Belassens des Haars 1 Minute oder mehr und 30 Minuten oder weniger beträgt.

17. Haarbehandlungsverfahren gemäß einem der Ansprüche 1 bis 16, wobei das Verfahren nicht einen Schritt des Auftragens eines Haarbehandlungsmittels mit einem pH-Wert von 12 bis 14 auf das Haar umfasst.

18. Haarbehandlungsverfahren, umfassend die folgenden Schritte (i-a) bis (iv'):
Schritt (i-a): ein Schritt des Auftragens eines einteiligen Haarbehandlungsmittels mit einem pH-Wert von 2 oder mehr und 7,5 oder weniger, das die folgenden Komponenten (A), (B) und (D) enthält und im Wesentlichen kein Haarreduktionsmittel enthält:
(A) 1 Massen-% oder mehr und 10 Massen-% oder weniger mindestens einer Verbindung, ausgewählt aus der Gruppe, bestehend aus Glycylglycin oder einem Salz davon, Glycylglycylglycin oder einem Salz davon, einem Polyglycerin mit einem durchschnittlichen Polymerisationsgrad von 2 oder mehr und 4 oder weniger und einem Alkylglycerylether mit einer Alkylgruppe mit 8 oder mehr und 18 oder weniger Kohlenstoffatomen
(B) 1 Massen-% oder mehr und 15 Massen-% oder weniger einer Carbonsäure mit einem anorganischen Wert von 260 oder mehr und 400 oder weniger und einem organischen Wert von 60 oder mehr und 180 oder weniger oder eines Salzes der Carbonsäure, wobei die Carbonsäure aus der Gruppe, bestehend aus einer Carbonsäure, dargestellt durch die folgende Formel (B-1), Äpfelsäure, Bernsteinsäure und Milchsäure, ausgewählt ist 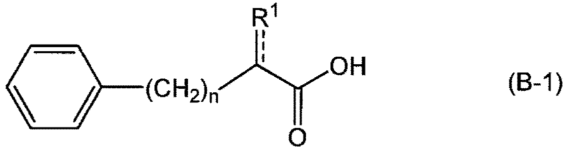 worin
R¹ für ein Wasserstoffatom, ein Sauerstoffatom oder eine Hydroxylgruppe steht;
wenn R¹ ein Sauerstoffatom ist, die gestrichelte Linie eine Doppelbindung anzeigt;
n für eine ganze Zahl von 0 bis 3 steht; und
die Phenylgruppe und die Methylenkette teilweise mit einer oder mehreren Hydroxylgruppen substituiert sein können;
(D) 0,5 Massen-% oder mehr und 3 Massen-% oder weniger eines Verdickungsmittels
Schritt (ii): ein Schritt des Belassens des Haars bei 15°C oder mehr und 60°C oder weniger für 15 Sekunden oder mehr und 45 Minuten oder weniger, nach Schritt (i-a);
Schritt (iii): ein Schritt des Auftragens eines Haarbehandlungsmittels mit einem pH-Wert von 3 oder mehr und 8 oder weniger, das die folgenden Komponenten (C), (D) und (E) und Wasser enthält und im Wesentlichen kein Haarreduktionsmittel enthält, auf das Haar nach Schritt (ii) :
(C) 1 Massen-% oder mehr und 15 Massen-% oder weniger mindestens einer, ausgewählt aus der Gruppe, bestehend aus 2-Naphthalinsulfonsäure, 1-Naphthalinsulfonsäure, p-Toluolsulfonsäure, Xylolsulfonsäure, Hydroxymethoxybenzophenonsulfonsäure und deren Salzen
(D) 0,5 Massen-% oder mehr und 3 Massen-% oder weniger eines Verdickungsmittels
(E) 4 Massen-% oder mehr und 15 Massen-% oder weniger eines aromatischen Alkohols;
Schritt (iv): ein Schritt des Belassens des Haars bei 15°C oder mehr und 60°C oder weniger für 3 Minuten oder mehr und 45 Minuten oder weniger, nach Schritt (iii);
Schritt (iv'): ein Schritt des Ausspülens des Haars nach Schritt (iv).

19. Haarbehandlungsverfahren, umfassend die folgenden Schritte (i-b) bis (iv'):
Schritt (i-b): ein Schritt des Mischens eines ersten Teils und eines zweiten Teils eines mehrteiligen Haarbehandlungsmittels, das die folgende Komponente (A) als den ersten Teil und die folgende Komponente (B) als den zweiten Teil enthält oder das die folgende Komponente (A) als den zweiten Teil und die folgende Komponente (B) als den ersten Teil enthält, das die folgende Komponente (D) in dem ersten Teil und/oder in dem zweiten Teil enthält und das im Wesentlichen kein Haarreduktionsmittel enthält, und des Auftragens der resultierenden Mischung mit einem pH-Wert von 2 oder mehr und 7,5 oder weniger auf das Haar:
(A) 1 Massen-% oder mehr und 10 Massen-% oder weniger mindestens einer Verbindung, ausgewählt aus der Gruppe bestehend aus Glycylglycin oder einem Salz davon, Glycylglycylglycin oder einem Salz davon, einem Polyglycerin mit einem durchschnittlichen Polymerisationsgrad von 2 oder mehr und 4 oder weniger und einem Alkylglycerylether mit einer Alkylgruppe mit 8 oder mehr und 18 oder weniger Kohlenstoffatomen
(B) 1 Massen-% oder mehr und 15 Massen-% oder weniger einer Carbonsäure mit einem anorganischen Wert von 260 oder mehr und 400 oder weniger und einem organischen Wert von 60 oder mehr und 180 oder weniger oder eines Salzes der Carbonsäure, wobei die Carbonsäure aus der Gruppe, bestehend aus einer Carbonsäure, dargestellt durch die folgende Formel (B-1), Äpfelsäure, Bernsteinsäure und Milchsäure, ausgewählt ist 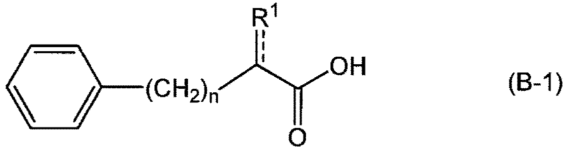 worin
R¹ für ein Wasserstoffatom, ein Sauerstoffatom oder eine Hydroxylgruppe steht;
wenn R¹ ein Sauerstoffatom ist, die gestrichelte Linie eine Doppelbindung anzeigt;
n für eine ganze Zahl von 0 bis 3 steht; und
die Phenylgruppe und die Methylenkette teilweise mit einer oder mehreren Hydroxylgruppen substituiert sein können
(D) 0,5 Massen-% oder mehr und 3 Massen-% oder weniger eines Verdickungsmittels,
Schritt (ii): ein Schritt des Belassens des Haars bei 15°C oder mehr und 60°C oder weniger für 15 Sekunden oder mehr und 45 Minuten oder weniger, nach Schritt (i-b);
Schritt (iii): ein Schritt des Auftragens eines Haarbehandlungsmittels mit einem pH-Wert von 3 oder mehr und 8 oder weniger, das die folgenden Komponenten (C), (D), (E) und Wasser enthält und im Wesentlichen kein Haarreduktionsmittel enthält, auf das Haar nach Schritt (ii) :
(C) 1 Massen-% oder mehr und 15 Massen-% oder weniger mindestens einer, ausgewählt aus der Gruppe, bestehend aus 2-Naphthalinsulfonsäure, 1-Naphthalinsulfonsäure, p-Toluolsulfonsäure, Xylolsulfonsäure, Hydroxymethoxybenzophenonsulfonsäure und deren Salzen
(D) 0,5 Massen-% oder mehr und 3 Massen-% oder weniger eines Verdickungsmittels;
(E) 4 Massen-% oder mehr und 15 Massen-% oder weniger eines aromatischen Alkohols;
Schritt (iv): ein Schritt des Belassens des Haars bei 15°C oder mehr und 60°C oder weniger für 3 Minuten oder mehr und 45 Minuten oder weniger nach Schritt (iii);
Schritt (iv'): ein Schritt des Ausspülens des Haars nach Schritt (iv).

20. Haarbehandlungsverfahren, umfassend die folgenden Schritte (i-c-1) bis (iv'):
Schritt (i-c-1): ein Schritt des Auftragens eines ersten Teils eines mehrteiligen Haarbehandlungsmittels vom Typ der sequentiellen Anwendung mit einem pH-Wert von 2 oder mehr und 7,5 oder weniger, der die folgende Komponente (A) enthält und im Wesentlichen kein Haarreduktionsmittel enthält, auf das Haar:
(A) 1 Massen-% oder mehr und 10 Massen-% oder weniger mindestens einer Verbindung, ausgewählt aus der Gruppe, bestehend aus Glycylglycin oder einem Salz davon, Glycylglycylglycin oder einem Salz davon, einem Polyglycerin mit einem durchschnittlichen Polymerisationsgrad von 2 oder mehr und 4 oder weniger und einem Alkylglycerylether mit einer Alkylgruppe mit 8 oder mehr und 18 oder weniger Kohlenstoffatomen;
Schritt (i-cx): ein Schritt des Belassens des Haars bei 15°C oder mehr und 60°C oder weniger für 15 Sekunden oder mehr und 30 Minuten oder weniger, nach Schritt (i-c) ;
Schritt (i-c-2): ein Schritt des Auftragens eines zweiten Teils des mehrteiligen Haarbehandlungsmittels vom Typ der sequentiellen Anwendung mit einem pH-Wert von 3,5 oder mehr und 8 oder weniger, der die folgenden Komponenten (B) und (D) enthält und im Wesentlichen kein Haarreduktionsmittel enthält, nach Schritt (i-cx):
(B) 1 Massen-% oder mehr und 15 Massen-% oder weniger einer Carbonsäure mit einem anorganischen Wert von 260 oder mehr und 400 oder weniger und einem organischen Wert von 60 oder mehr und 180 oder weniger oder eines Salzes der Carbonsäure, wobei die Carbonsäure aus der Gruppe, bestehend aus einer Carbonsäure, dargestellt durch die folgende Formel (B-1), Äpfelsäure, Bernsteinsäure und Milchsäure, ausgewählt ist 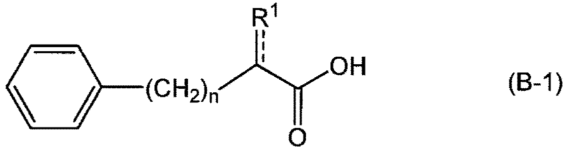 worin
R¹ für ein Wasserstoffatom, ein Sauerstoffatom oder eine Hydroxylgruppe steht;
wenn R¹ ein Sauerstoffatom ist, die gestrichelte Linie eine Doppelbindung anzeigt;
n für eine ganze Zahl von 0 bis 3 steht; und
die Phenylgruppe und die Methylenkette teilweise mit einer oder mehreren Hydroxylgruppen substituiert sein können
(D) 0,5 Massen-% oder mehr und 3 Massen-% oder weniger eines Verdickungsmittels
Schritt (ii): ein Schritt des Belassens des Haars bei 15°C oder mehr und 60°C oder weniger für 15 Sekunden oder mehr und 45 Minuten oder weniger, nach Schritt (i-c-2);
Schritt (iii): ein Schritt des Auftragens eines Haarbehandlungsmittels mit einem pH-Wert von 3 oder mehr und 8 oder weniger, das die folgenden Komponenten (C), (D), (E) und Wasser enthält und im Wesentlichen kein Haarreduktionsmittel enthält, nach Schritt (ii):
(C) 1 Massen-% oder mehr und 15 Massen-% oder weniger mindestens einer, ausgewählt aus der Gruppe, bestehend aus 2-Naphthalinsulfonsäure, 1-Naphthalinsulfonsäure, p-Toluolsulfonsäure, Xylolsulfonsäure, Hydroxymethoxybenzophenonsulfonsäure und deren Salzen
(D) 0,5 Massen-% oder mehr und 3 Massen-% oder weniger eines Verdickungsmittels
(E) 4 Massen-% oder mehr und 15 Massen-% oder weniger eines aromatischen Alkohols;
Schritt (iv): ein Schritt des Belassens des Haars bei 15°C oder mehr und 60°C oder weniger für 3 Minuten oder mehr und 45 Minuten oder weniger, nach Schritt (iii);
Schritt (iv'): ein Schritt des Ausspülens des Haars nach Schritt (iv).

## Revendications

1. Procédé de traitement capillaire comprenant les étapes suivantes (i) à (iii) :
étape (i) : une étape d'application sur les cheveux d'un agent de traitement capillaire contenant les composants (A) et (B) suivants :
(A) au moins un composé choisi dans le groupe constitué d'un dérivé de glycylglycine représenté par la formule (1) ou un sel de celui-ci, une polyglycérine ayant un degré moyen de polymérisation de 2 ou plus et de 6 ou moins, et un éther glycérique d'alkyle ayant un groupe alkyle ayant 6 atomes de carbone ou plus et 40 atomes de carbone ou moins, 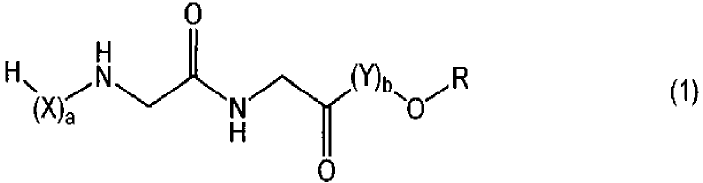 dans laquelle X représente un groupe hydrocarbure divalent ayant 1 à 4 atomes de carbone et facultativement substitué par un ou plusieurs groupes hydroxyle ou un résidu d'acide aminé, et
Y représente un résidu d'acide aminé ou un groupe divalent représenté par la formule (2) : 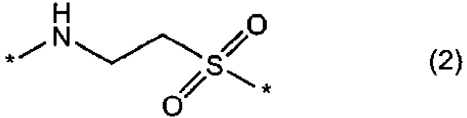 dans laquelle -* représente une liaison à un groupe carbonyle adjacent ou un atome d'oxygène,
R représente un atome d'hydrogène ou un groupe d'hydrocarbure monovalent ayant 1 à 4 atomes de carbone et facultativement substitué par un ou plusieurs groupes hydroxyle, et
a et b représentent 0 ou 1, à la condition que si a et b représentent simultanément 1, alors X ne représente pas un résidu d'acide aminé ;
(B) un acide carboxylique ayant une valeur inorganique de 250 ou plus et 450 ou moins et une valeur organique de 50 ou plus et 250 ou moins, ou un sel de l'acide carboxylique, dans lequel l'acide carboxylique est choisi dans le groupe constitué d'un acide carboxylique représenté par la formule (B-1) suivante, un acide malique, un acide succinique, et un acide lactique 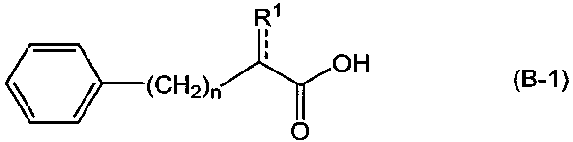 dans laquelle
R¹ représente un atome d'hydrogène, un atome d'oxygène, ou un groupe hydroxyle ;
lorsque R¹ est un atome d'oxygène, la ligne en pointillé indique une double liaison ;
n représente un nombre entier de 0 à 3 ; et
le groupe phényle et la chaîne méthylène peuvent être partiellement substitués par un ou plusieurs groupes hydroxyle ;
étape (ii) : une étape consistant à laisser les cheveux reposer à 15 °C ou plus et 100 °C ou moins pendant 15 secondes ou plus et 60 minutes ou moins, après l'étape (i) ; et
étape (iii) : une étape d'application sur les cheveux d'un agent de traitement capillaire contenant le composant (C) suivant, après l'étape (ii) :
(C) un acide sulfonique aromatique ayant un poids moléculaire de 300 ou moins, ou un sel de celui-ci, dans lequel le procédé ne comprend pas d'application d'un agent de traitement capillaire contenant un agent de réduction.

2. Procédé de traitement capillaire selon la revendication 1, dans lequel l'agent de traitement capillaire contenant les composants (A) et (B) est composé d'une composition unique contenant les composants (A) et (B), ou est composé à la fois d'une composition contenant le composant (A) et d'une composition contenant le composant (B) en tant que constituants.

3. Procédé de traitement capillaire selon la revendication 1 ou la revendication 2, dans lequel l'agent de traitement capillaire contenant les composants (A) et (B) est un agent de traitement capillaire en une partie contenant les composants (A) et (B).

4. Procédé de traitement capillaire selon la revendication 1 ou la revendication 2, dans lequel l'agent de traitement capillaire contenant les composants (A) et (B) est un agent de traitement capillaire en plusieurs parties comprenant une première partie contenant le composant (A) et une seconde partie contenant le composant (B) ou un agent de traitement capillaire en plusieurs parties comprenant une première partie contenant le composant (B) et une seconde partie contenant le composant (A).

5. Procédé de traitement capillaire selon la revendication 4, dans lequel l'étape (i) est effectuée par l'application de la première partie sur les cheveux, et ensuite l'application de la seconde partie sur les cheveux.

6. Procédé de traitement capillaire selon la revendication 5, dans lequel l'étape (i) comprend en outre l'étape (i-cx) après l'application de la première partie sur les cheveux et avant l'application de la seconde partie sur les cheveux :
étape (i-cx) : une étape consistant à laisser les cheveux reposer à 15 °C ou plus et 100 °C ou moins pendant 15 secondes ou plus et 60 minutes ou moins.

7. Procédé de traitement capillaire selon l'une quelconque des revendications 1 à 6, comprenant une étape de rinçage des cheveux après l'étape (iii), et comprenant en outre l'étape (iv) entre l'étape (iii) et l'étape de rinçage des cheveux :
étape (iv) : une étape consistant à laisser les cheveux, sur lesquels l'agent de traitement capillaire contenant le composant (C) est appliqué, reposer à 15 °C ou plus et 100 °C ou moins pendant 1 minute ou plus et 60 minutes ou moins.

8. Procédé de traitement capillaire selon l'une quelconque des revendications 2 à 7, dans lequel la teneur d'un solvant (F) organique représenté par la formule (F-1) suivante dans la composition contenant le composant (B) est inférieure à 5 % en masse,
R²-(OCH₂CH₂)_{q}-R³ (F-1)
dans laquelle R² représente un atome d'hydrogène ou un groupe alkyle ayant 1 atome de carbone ou plus et 5 atomes de carbone ou moins ; R³ représente un atome d'hydrogène ou un ou plusieurs groupes hydroxyle ; et q représente un nombre entier de 0 ou plus et 5 ou moins, à condition que si q est 0, alors R² et R³ ne sont pas des atomes d'hydrogène.

9. Procédé de traitement capillaire selon l'une quelconque des revendications 2 à 8, dans lequel la teneur d'un alcool aromatique (E) dans la composition contenant le composant (B) est inférieure à 5 % en masse.

10. Procédé de traitement capillaire selon l'une quelconque des revendications 2 à 9, dans lequel la composition contenant le composant (A) et/ou le composant (B) contient en outre un épaississant en tant que composant (D).

11. Procédé de traitement capillaire selon l'une quelconque des revendications 2 à 10, dans lequel le pH de la composition contenant le composant (B) à 25 °C est de 2 ou plus et 10 ou moins, pourvu que la composition ne contienne pas le composant (A).

12. Procédé de traitement capillaire selon la revendication 11, dans lequel le pH de la composition contenant le composant (B) à 25 °C est de 5 ou plus et 8 ou moins, pourvu que la composition ne contienne pas le composant (A).

13. Procédé de traitement capillaire selon l'une quelconque des revendications 2 à 10, dans lequel le pH de la composition contenant les composants (A) et (B) à 25 °C est 2 ou plus et 10 ou moins.

14. Procédé de traitement capillaire selon l'une quelconque des revendications 1 à 13, dans lequel l'agent de traitement capillaire contenant le composant (C) contient en outre un épaississant en tant que composant (D) et un alcool aromatique en tant que composant (E), et la teneur du composant (D) est de 0,1% en masse à 0,5 % en masse.

15. Procédé de traitement capillaire selon l'une quelconque des revendications 7 à 14, dans lequel la température dans l'étape (iv) à laquelle les cheveux reposent est de 15 °C ou plus et inférieure à 30 °C.

16. Procédé de traitement capillaire selon l'une quelconque des revendications 7 à 14, dans lequel la température dans l'étape (iv) à laquelle les cheveux reposent est de 30 °C ou plus et 100 °C ou moins, et la durée de repos des cheveux est de 1 minute ou plus et 30 minutes ou moins.

17. Procédé de traitement capillaire selon l'une quelconque des revendications 1 à 16, dans lequel le procédé ne comprend pas d'étape d'application d'un agent de traitement capillaire de pH 12 à 14 sur les cheveux.

18. Procédé de traitement capillaire comprenant les étapes (i-a) à (iv') suivantes :
étape (i-a) : une étape d'application d'un agent de traitement capillaire en une partie de pH 2 ou plus et 7,5 ou moins contenant les composants (A), (B) et (D) suivants et ne contenant sensiblement pas d'agent de réduction capillaire :
(A) 1 % en masse ou plus et 10 % en masse ou moins d'au moins un composé choisi dans le groupe constitué de glycylglycine ou d'un sel de celle-ci, de glycylglycylglycine ou d'un sel de celle-ci, d'une polyglycérine ayant un degré moyen de polymérisation de 2 ou plus et 4 ou moins et un éther glycérique d'alkyle ayant un groupe alkyle ayant 8 atomes de carbone ou plus et 18 atomes de carbone ou moins
(B) 1 % en masse ou plus et 15 % en masse ou moins d'un acide carboxylique ayant une valeur inorganique de 260 ou plus et 400 ou moins et une valeur organique de 60 ou plus et 180 ou moins, ou un sel de l'acide carboxylique, dans lequel l'acide carboxylique est choisi dans le groupe constitué d'un acide carboxylique représenté par la formule (B-1) suivante, un acide malique, un acide succinique, et un acide lactique 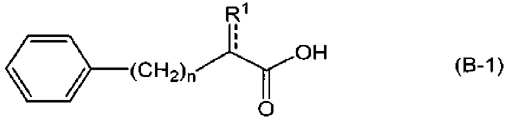 dans laquelle
R¹ représente un atome d'hydrogène, un atome d'oxygène, ou un groupe hydroxyle ;
lorsque R¹ est un atome d'oxygène, la ligne en pointillé indique une double liaison ;
n représente un nombre entier de 0 à 3 ; et
le groupe phényle et la chaîne méthylène peuvent être partiellement substitués par un ou plusieurs groupes hydroxyle ;
(D) 0,5 % en masse ou plus et 3 % en masse ou moins d'un épaississant
étape (ii) : une étape consistant à laisser les cheveux reposer à 15 °C ou plus et 60 °C ou moins pendant 15 secondes ou plus et 45 minutes ou moins, après l'étape (i-a) ;
étape (iii) : une étape d'application sur les cheveux d'un agent de traitement capillaire de pH 3 ou plus et 8 ou moins contenant les composants suivants (C), (D) et (E) et de l'eau et ne contenant sensiblement pas d'agent de réduction capillaire, après l'étape (ii) :
(C) 1 % en masse ou plus et 15 % en masse ou moins d'au moins un choisi dans le groupe constitué d'acide 2-naphthalène sulfonique, acide 1-naphthalène sulfonique, acide p-toluènesulfonique, acide xylène sulfonique, acide hydroxyméthoxy benzophénone sulfonique, et des sels de ceux-ci
(D) 0,5 % en masse ou plus et 3 % en masse ou moins d'un épaississant
(E) 4 % en masse ou plus et 15 % en masse ou moins d'un alcool aromatique ;
étape (iv) : une étape consistant à laisser les cheveux reposer à 15 °C ou plus et 60 °C ou moins pendant 3 minutes ou plus et 45 minutes ou moins, après l'étape (iii) ;
étape (iv') : une étape de rinçage des cheveux, après l'étape (iv).

19. Procédé de traitement capillaire comprenant les étapes (i-b) à (iv') suivantes :
étape (i-b) : une étape de mélange d'une première partie et d'une seconde partie d'un agent de traitement capillaire en plusieurs parties, qui contient le composant (A) suivant en tant que première partie et le composant (B) suivant en tant que seconde partie ou qui contient le composant (A) suivant en tant que seconde partie et le composant (B) suivant en tant que première partie, qui contient le composant (D) suivant dans la première partie et/ou la seconde partie et qui ne contient sensiblement pas d'agent de réduction capillaire et une étape d'application sur les cheveux du mélange résultant, ayant un pH de 2 ou plus et 7,5 ou moins :
(A) 1 % en masse ou plus et 10 % en masse ou moins d'au moins un composé choisi dans le groupe constitué de glycylglycine ou d'un sel de celle-ci, de glycylglycylglycine ou d'un sel de celle-ci, d'une polyglycérine ayant un degré moyen de polymérisation de 2 ou plus et 4 ou moins et un éther glycérique d'alkyle ayant un groupe alkyle ayant 8 atomes de carbone ou plus et 18 atomes de carbone ou moins
(B) 1 % en masse ou plus et 15 % en masse ou moins d'un acide carboxylique ayant une valeur inorganique de 260 ou plus et 400 ou moins et une valeur organique de 60 ou plus et 180 ou moins, ou un sel de l'acide carboxylique, dans lequel l'acide carboxylique est choisi dans le groupe constitué d'un acide carboxylique représenté par la formule (B-1) suivante, un acide malique, un acide succinique, et un acide lactique 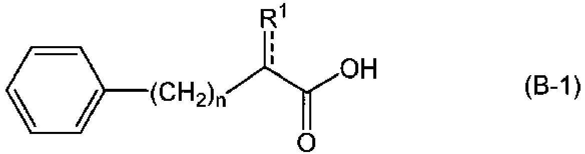 dans laquelle
R¹ représente un atome d'hydrogène, un atome d'oxygène, ou un groupe hydroxyle ;
lorsque R¹ est un atome d'oxygène, la ligne en pointillé indique une double liaison ;
n représente un nombre entier de 0 à 3 ; et
le groupe phényle et la chaîne méthylène peuvent être partiellement substitués par un ou plusieurs groupes hydroxyle
(D) 0,5 % en masse ou plus et 3 % en masse ou moins d'un épaississant,
étape (ii) : une étape consistant à laisser les cheveux reposer à 15 °C ou plus et 60 °C ou moins pendant 15 secondes ou plus et 45 minutes ou moins, après l'étape (i-b) ;
étape (iii) : une étape d'application sur les cheveux d'un agent de traitement capillaire de pH 3 ou plus et 8 ou moins contenant les composants (C), (D), (E) suivants et de l'eau et ne contenant sensiblement pas d'agent de réduction capillaire, après l'étape (ii) :
(C) 1 % en masse ou plus et 15 % en masse ou moins d'au moins un choisi dans le groupe constitué d'acide 2-naphthalène sulfonique, acide 1-naphthalène sulfonique, acide p-toluènesulfonique, acide xylène sulfonique, acide hydroxyméthoxy benzophénone sulfonique, et des sels de ceux-ci
(D) 0,5 % en masse ou plus et 3 % en masse ou moins d'un épaississant ;
(E) 4 % en masse ou plus et 15 % en masse ou moins d'un alcool aromatique ;
étape (iv) : une étape consistant à laisser les cheveux reposer à 15 °C ou plus et 60 °C ou moins pendant 3 minutes ou plus et 45 minutes ou moins, après l'étape (iii) ;
étape (iv') : une étape de rinçage des cheveux après l'étape (iv).

20. Procédé de traitement capillaire comprenant les étapes (i-c-1) à (iv') suivantes :
étape (i-c-1) : une étape d'application sur les cheveux d'une première partie d'un agent de traitement capillaire en plusieurs parties de type à application séquentielle de pH 2 ou plus et 7,5 ou moins contenant le composant (A) suivant et ne contenant sensiblement pas d'agent de réduction capillaire :
(A) 1 % en masse ou plus et 10 % en masse ou moins d'au moins un composé choisi dans le groupe constitué de glycylglycine ou d'un sel de celle-ci, de glycylglycylglycine ou d'un sel de celle-ci, d'une polyglycérine ayant un degré moyen de polymérisation de 2 ou plus et 4 ou moins et un éther glycérique d'alkyle ayant un groupe alkyle ayant 8 atomes de carbone ou plus et 18 atomes de carbone ou moins ;
étape (i-cx) : une étape consistant à laisser les cheveux reposer à 15 °C ou plus et 60 °C ou moins pendant 15 secondes ou plus et 30 minutes ou moins, après l'étape (i-c) ;
étape (i-c-2) : une étape d'application d'une seconde partie de l'agent de traitement capillaire en plusieurs parties de type à application séquentielle de pH 3,5 ou plus et 8 ou moins contenant les composants (B) et (D) suivants et ne contenant sensiblement pas d'agent de réduction capillaire, après l'étape (i-cx) :
(B) 1 % en masse ou plus et 15 % en masse ou moins d'un acide carboxylique ayant une valeur inorganique de 260 ou plus et 400 ou moins et une valeur organique de 60 ou plus et 180 ou moins, ou un sel de l'acide carboxylique, dans lequel l'acide carboxylique est choisi dans le groupe constitué d'un acide carboxylique représenté par la formule (B-1) suivante, un acide malique, un acide succinique, et un acide lactique 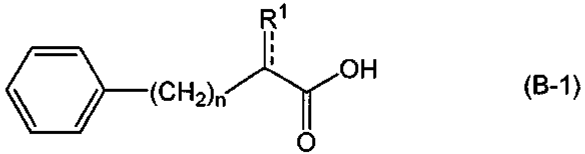 dans lequel
R¹ représente un atome d'hydrogène, un atome d'oxygène, ou un groupe hydroxyle ;
lorsque R¹ est un atome d'oxygène, la ligne en pointillé indique une double liaison ;
n représente un entier de 0 à 3 ; et
le groupe phényle et la chaîne méthylène peuvent être partiellement substitués par un ou plusieurs groupes hydroxyle
(D) 0,5 % en masse ou plus et 3 % en masse ou moins d'un épaississant
étape (ii) : une étape consistant à laisser les cheveux reposer à 15 °C ou plus et 60 °C ou moins pendant 15 secondes ou plus et 45 minutes ou moins, après l'étape (i-c-2) ;
étape (iii) : une étape d'application d'un agent de traitement capillaire de pH 3 ou plus et 8 ou moins contenant les composants (C), (D), (E) suivants et de l'eau et ne contenant sensiblement pas d'agent de réduction capillaire, après l'étape (ii) :
(C) 1 % en masse ou plus et 15 % en masse ou moins d'au moins un choisi dans le groupe constitué d'acide 2-naphthalène sulfonique, acide 1-naphthalène sulfonique, acide p-toluènesulfonique, acide xylène sulfonique, acide hydroxyméthoxy benzophénone sulfonique, et des sels de ceux-ci
(D) 0,5 % en masse ou plus et 3 % en masse ou moins d'un épaississant
(E) 4 % en masse ou plus et 15 % en masse ou moins d'un alcool aromatique ;
étape (iv) : une étape consistant à laisser les cheveux reposer à 15 °C ou plus et 60 °C ou moins pendant 3 minutes ou plus et 45 minutes ou moins, après l'étape (iii) ;
étape (iv') : une étape de rinçage des cheveux, après l'étape (iv).
